# EUROPEAN PATENT APPLICATION

(11) **EP 1 394 141 A1**
(43) Date of publication of application: **03.03.2004**
(21) Application number: 03022652.6
(22) Date of filing: 08.01.1998
(51) Int. Cl.: C07C 2/86, C07C 41/30, C07C 51/14, C07C 67/38, C07C 51/487

(54) **Preparation of aryl olefins**

(30) Priority: 08.01.1997 US 780308
(62) Divisional of application: 02002018.6
(71) Applicant: ALBEMARLE CORPORATION, Baton Rouge, LA 70801-1765 (US)
(72) Inventor: Lin, Ronny W., Baton Rouge LA 70816 (US); Herndon, Robert C., Baton Rouge LA 70816 (US); Allen, Robert H., Baton Rouge LA 70808 (US); Chockalingham, Kannappan, Baton Rouge LA 70817 (US); Focht, Gary D., Baton Rouge LA 70808 (US)
(74) Representative: UEXKÜLL & STOLBERG

(57) **Abstract**

The invention is directed to a process for preparing an arylolefin or a substituted arylolefin by palladium-catalyzed arylation of vinylolefin with an aryl halide or substituted aryl halide in a liquid polar organic solvent containing one or more secondary or tertiary amines as hydrogen halide acceptor, mixing at least a portion of the reaction mixture with diluted aqueous acid, and separting the formed organic and aqueous phases.

## Description

### TECHNICAL FIELD

This invention relates to the synthesis of certain carboxylic acids, or derivatives thereof, such as salts or esters.

### BACKGROUND

The palladium-catalyzed vinylation of organic halides provides a very convenient method for forming carbon-carbon bonds at unsubstituted vinylic positions. The reaction, reported by Heck *(Palladium Reagents* in *Organic Synthesis.* Academic Press, Canada 1985) can be used to prepare fine organics, pharmaceuticals, and specialty monomers. For example, the reaction allows a one-step synthesis of substituted styrenes from aryl bromides and is an excellent method for preparation of a wide variety of styrene derivatives. Heitz et al., *Makromol. Chem.,* **189**, 119 (1968).

Vinyl toluenes have been reported as the product of a homogeneous palladium-catalyzed coupling of ethylene with bromotoluenes. The reaction is performed in a two-phase solvent system composed of N,N-dimethyl formamide and water. R. A. DeVries et al., *Organometallics,* **13**, 2405 (1994).

U.S. Pat. Nos. 5,136,069 and 5,243,068 to R. A. DeVries et al. describe preparation of vinylically-unsaturated compounds by reaction of a halogenated organic compound with a hydrolytically-stable, vinylically-unsaturated precursor compound in the presence of (a) a homogeneous zerovalent palladium catalyst complex, (b) an inorganic hydrogen halide acceptor and (c) a diluent which is either water or an aqueous solution containing up to 95 % by volume of organic solvent.

Arylation of propylene, ethylene, styrene, and methyl acrylate with iodobenzene was found to be catalyzed by metallic palladium in methanol to give methylstyrene, styrene, t-stilbene, and methyl cinnamate, respectively. Their yields and selectivities increased significantly by the addition of excess potassium acetate as an acceptor of hydriodic acid formed. Mori et al., *Bull. Chem. Soc., Japan,* **46,** 1505 (1973).

A variety of styrene derivatives and 3-vinylpyridine were prepared in moderate to good yields by the palladium-tri-o-tolylphosphine catalyzed reaction of ethylene with aryl bromides or 3-bromopyridine, respectively, (Plevyak et al., *J. Org. Chem.,* **43**, 2454 (1970).

Alper et al. in *J. Chem Soc. Chem. Comm.,* **1983**. 1270-1271, discloses that alkenes can react with carbon monoxide, water, hydrochloric acid and a mixture of palladium and copper to produce the hydrocarboxylated branched chain carboxylic acid. Oxygen is necessary to succeed in the reaction.

A process for preparing the branched chain carboxylic acid ibuprofen is described in Japanese Patent Application (Kokai) No. 59-10545 (Mitsubishi Petrochemical, published January, 1984), which teaches that ibuprofen can be prepared by reacting p-isobutylstyrene with carbon monoxide and water or an alcohol in the presence of a palladium(II) catalyst and a peroxide. e.g., cumyl hydroperoxide.

A process for preparing aryl substituted aliphatic carboxylic acids or their alkyl esters is disclosed in U. S. Patent No. 5.315,026. A 1-aryl substituted olefin is reacted with carbon monoxide in the presence of water or an alcohol at a temperature between 25°C and 200°C. A mixture useful as a catalyst is a palladium compound and a copper compound with at least one acid-stable ligand. Ligands which may be used include monodentate or multidentate electron-donating substances such as those containing elements P, N, or O, and those containing multiple bonds such as olefinic compounds. Examples of such acid-stable ligands are trihydrocarbylphosphines, including trialkyl-and triarylphosphines, such as tri-n-butyl-, tricyclohexyl-, and triphenylphosphine: lower alkyl and aryl nitriles, such as benzonitrile and n-propionitrile: ligands containing pi-electrons, such as an allyl compound or 1,5-cyclooctadiene: piperidine, piperazine, trichloro-stannate(II), and acetylacetonate.

U.S. Pat. No. 5,536,870 describes the preparation of substituted olefins by the palladium-catalyzed coupling of vinyl or substituted vinyl compounds with organic halides, and also the formation of carboxylic acids and esters from such substituted olefins. The substituted olefinic compounds are formed by reacting an organic halide with a vinyl or substituted vinyl compound in the presence of a catalytically effective amount of palladium or a salt of palladium having a valence of 1 or 2, and a tertiary phosphine ligand such as neomenthyldiphenylphosphine. This reaction is carried out in the presence or absence of a solvent such as acetonitrile, tetrahydrofuran. dioxane, or dimethylformamide. An important utility of the substituted olefins formed in this manner is the subsequent conversion of such substituted olefins to carboxylic acids or derivatives thereof such as salts or esters (e.g., profen compounds) by carbonylation with carbon monoxide using catalytic systems and reaction conditions described in U.S. Pat. No. 5,536,870.

### SUMMARY OF THE INVENTION

This invention provides, *inter alia,* process technology enabling the efficient large-scale production of certain aromatically-substituted aliphatic carboxylic acids and their acid derivatives such as salts or esters. including profen-type compounds, which are well-known analgesic or anti-inflammatory agents.

In accordance with one of the embodiments of this invention, there is provided a process which comprises:
a) conducting a palladium-catalyzed arylation of an olefin with aryl halide and/or substituted aryl halide in a liquid medium formed from (i) one or more liquid polar organic solvent/diluents. and (ii) one or more secondary or tertiary amines that (1) boil(s) below the boiling temperature of the solvent/diluent if only one solvent/diluent is used or (2) that boil(s) below the boiling temperature of at least one, but not necessarily all, of the polar solvent/diluents used in forming said medium if more than one solvent/diluent is used, to form a reaction mixture comprising olefinically-substituted aromatic compound, amine-hydrohalide and one or more polar organic solvents;
b) mixing (i) a concentrated aqueous solution of inorganic base having a base strength greater than the base strength of the one or more secondary or tertiary amines, with (ii) at least a portion of the reaction mixture to convert amine-hydrohalide therein to free amine and alkali metal halide, and to form (i) an aqueous phase containing dissolved alkali metal halide, and (ii) an organic phase comprising olefinically-substituted aromatic compound, one or more polar organic solvents and free amine;
c) separating the foregoing phases from each other;
d) distilling off substantially all of the amine from the organic phase under low temperature and pressure conditions that suppress thermal oligomerization of the olefinically-substituted aromatic compound contained in the residual liquid phase, to thereby form a distilland composed predominately of olefinically-substituted aromatic compound and one or more polar organic solvents; and
e) conducting a palladium-catalyzed carboxylation of at least a portion of said olefinically-substituted aromatic compound with carbon monoxide and water and/or alcohol in a liquid medium comprising at least a portion of said distilland.

The relatively high specific gravity and salt concentration of the aqueous phase formed in b) greatly facilitates the phase separation of c), and enables efficient recovery of amine and recycle of solvent.

Preferably liquid organic makeup solvent, is mixed with the liquid medium during or after the distillation of d) whereby the liquid medium of e) further comprises at least a portion of both distilland and such makeup solvent.

Another embodiment is a process which comprises (a) forming a reaction product composition comprising arylolefin or a substituted arylolefin (e.g.. 6-methoxy-2-vinyl-naphthalene or 4-isobutylstyrene), and amine-hydrohalide in a liquid polar organic solvent medium by palladium-catalyzed arylation of a vinylolefin (e.g., ethylene) with an aryl halide and/or substituted aryl halide (e.g., 2-bromo-6-methoxynaphthalene or 4-bromo-isobutylbenzene), in a liquid polar organic solvent containing one or more secondary or tertiary amines as hydrogen halide. acceptor, and (b) mixing with such reaction product composition a concentrated aqueous solution of inorganic base having a base strength greater than that of the one or more secondary or tertiary amines, to thereby form (i) an organic phase containing such arylolefin or substituted arylolefin, and the one or more secondary or tertiary amines, and (ii) a lower aqueous phase containing dissolved inorganic salt such that said aqueous phase has a specific gravity of at least 1.08 grams per milliliter, when and if measured at 25°C, and (c) separating the phases from each other.

Still another embodiment is a process which comprises (A) forming a reaction product composition comprising arylcarboxylic acid or a substituted arylcarboxylic acid (e.g., racemic 2-(6-methoxy-2-naphthyl)propionic acid or 2-(4-isobutylphenyl)propionic acid) in a liquid polar organic solvent medium by palladium-catalyzed hydrocarboxylation of an arylolefin or substituted arylolefin (e.g., 6-methoxy-2-vinyl-naphthalene or 4-isobutylstyrene), in a liquid medium comprising polar organic solvent (preferably ketone), water, HCl, and at least one ether (e.g., THF), (B) mixing with such reaction product composition an aqueous solution of inorganic base (e.g.. 25 wt% aqueous NaOH, KOH), to thereby form a mixture containing an aqueous phase containing dissolved inorganic salt of such arylcarboxylic acid or a substituted arylcarboxylic, and before, during or after such mixing, distilling at least a substantial portion of the ether from the reaction mixture, such that a mixture of residual organic phase and such aqueous phase remain as a distillation residue (distilland or pot residue), (C) separating the phases from each other, (D) distilling residual organic impurities from the aqueous phase and, if necessary, adjusting the concentration of such aqueous phase to between 10 and 35 wt% solution by removal or addition of water, (E) washing the aqueous solution with substantially non-polar liquid organic solvent (preferably a paraffinic hydrocarbon solvent such as hexane or heptane, or an aromatic hydrocarbon solvent such as toluene or xylene), preferably at least twice, (F) mixing non-oxidizing mineral acid (e.g., sulfuric acid) with the aqueous phase in the presence of substantially non-polar liquid solvent to form (i) an organic phase composed of a solution of arylcarboxylic acid or a substituted arylcarboxylic acid in substantially non-polar liquid solvent and (ii) an aqueous phase, (G) separating the phases from each other, and (H) crystallizing arylcarboxylic acid or substituted arylcarboxylic acid from the substantially non-polar liquid solvent.

The above and other embodiments will be apparent from the ensuing description and appended claims.

### GLOSSARY OF TERMS

In the specification and claims hereof, and unless otherwise specified therein, the following terms have the following meanings:
"alkyl" means straight or branched chain alkyl having 1 to 20 carbon atoms and includes, for example, methyl, ethyl, propyl, isopropyl, butyl, isobutyl, secondary butyl, tertiary butyl, pentyl, isopentyl, neopentyl, hexyl, heptyl, octyl, 2-ethylhexyl, 1,1,3,3-tetramethylbutyl, nonyl, decyl, dodecyl, tetradecyl, hexadecyl, octadecyl and eicosyl, and "C₁ to C₆ alkyl" means alkyl with 1 to 6 carbon atoms;
"cycloalkyl" means cyclic alkyl having 3 to 7 carbon atoms and includes cyclopropyl, cyclobutyl, cyclopentyl, cyclohexyl, or cycloheptyl;
"substituted cycloalkyl" means cyclopropyl, cyclobutyl, cyclopentyl, cyclohexyl, or cycloheptyl substituted by at least one substituent selected from aroyl (as defined below), halogen (chlorine, bromine, fluorine or iodine), amino, nitro, hydroxy, alkyl, alkoxy (which means straight or branched chain alkoxy having 1 to 10 carbon atoms, and includes, for example, methoxy, ethoxy, propoxy, isopropoxy, butoxy, isobutoxy, secondary butoxy, tertiary butoxy, pentoxy, isopentoxy, hexyloxy, heptyloxy, octyloxy, nonyloxy and decyloxy), cycloalkyloxy including cyclopentyloxy, cyclohexyloxy and cycloheptyloxy, aryloxy including phenoxy and phenoxy substituted with halo, alkyl, or alkoxy, haloalkyl which means straight or branched chain alkyl having 1 to 8 carbon atoms which are substituted by at least one halogen, and includes, for example, chloromethyl, bromomethyl, fluoromethyl, iodomethyl, 2-chloroethyl, 2-bromoethyl, 2-fluoroethyl, 3-chloropropyl, 3-bromopropyl, 3-fluoropropyl, 4-chlorobutyl, 4-fluorobutyl, dichloromethyl, dibromomethyl, difluoromethyl, diiodomethyl, 2,2-dichloroethyl, 2,2-diibromoethyl, 2,2-difluoroethyl, 3,3-dichloropropyl, 3,3-difluoropropyl, 4,4-dichlorobutyl, 4,4-difluorobutyl, trichloromethyl, trifluoromethyl, 2,2,2-tri-fluoroethyl, 2,3,3-trifluoropropyl, 1,1,2,2-tetrafluoroethyl, or 2,2,3,3-tetrafluoropropyl;
"aryl" means phenyl, naphthyl, or biphenyl;
"substituted aryl" means phenyl, naphthyl, or biphenyl substituted by at least one substituent selected from aroyl (as defined below), halogen (chlorine, bromine, fluorine or iodine), amino, nitro, hydroxy, alkyl, alkoxy (which means straight or branched chain alkoxy having 1 to 10 carbon atoms, and includes, for example, methoxy, ethoxy, propoxy, isopro-poxy, butoxy, isobutoxy, secondary butoxy, tertiary butoxy, pentoxy, isopentoxy, hexyloxy, heptyloxy, octyloxy, nonyloxy and decyloxy), cycloalkyloxy including cyclopentyloxy, cyclohexyloxy and cycloheptyloxy, aryloxy including phenoxy and phenoxy substituted with halo, alkyl, or alkoxy, haloalkyl which means straight or branched chain alkyl having 1 to 8 carbon atoms which are substituted by at least one halogen, and includes, for example, chloromethyl, bromomethyl, fluoromethyl, iodomethyl, 2-chloroethyl, 2-bromoethyl, 2-fluoroethyl, 3-chloropropyl, 3-bromopropyl, 3-fluoropropyl, 4-chlorobutyl, 4-fluorobutyl, dichloromethyl, dibromomethyl, difluoromethyl, diiodomethyl, 2,2-dichloroethyl, 2,2-diibromoethyl, 2,2-difluoroethyl, 3,3-dichloropropyl, 3,3-difluoropropyl, 4,4-dichlorobutyl, 4,4-difluorobutyl, trichloromethyl, trifluoromethyl, 2,2,2-tri-fluoroethyl, 2,3,3-trifluoropropyl, 1,1,2,2-tetrafluoroethyl, or 2,2,3,3-tetrafluoropropyl;
"cycloalkylalkyl" means a straight or branched chain alkyl moiety having 1 to 8 carbon atoms that is substituted by a cycloalkyl group having 3 to 7 carbon atoms, and includes, for example, cyclopropylcarbinyl (i.e., carbinyl may also be termed methyl in this context), cyclobutylcarbinyl, cyclopentylcarbinyl, cyclohexylcarbinyl, cycloheptylmethyl, 2-cyclopropylethyl, 2-cyclopentylethyl, 2-cyclohexylethyl, 3-cyclopropylpropyl, 3-cyclo-pentylpropyl, 3-cyclohexylpropyl, 4-cyclopropylbutyl, 4-cyclopentylbutyl, 4-cyclohexylbutyl, 6-cyclopropylhexyl, or 6-cyclohexylhexyl;
"aralkyl" means a straight or branched chain alkyl moiety having 1 to 8 carbon atoms that is substituted by an aryl group or a substituted aryl group having 6 to 12 carbon atoms, and includes benzyl, 2-phenethyl, 2-methylbenzyl, 3-methylbenzyl, 4-methylbenzyl, 2,4-dimethylbenzyl, 2-(4-ethylphenyl)ethyl, or 3-(3-propylphenyl)propyl;
substituted aralkyl" means aralkyl substituted by at least one substituent selected from aroyl (as defined below), halogen (chlorine, bromine, fluorine or iodine), amino, nitro, hydroxy, alkyl, alkoxy (which means straight or branched chain alkoxy having 1 to 10 carbon atoms, and includes, for example, methoxy, ethoxy. propoxy, isopropoxy, butoxy, isobutoxy, secondary butoxy, tertiary butoxy, pentoxy, isopentoxy, hexyloxy, heptyloxy, octyloxy, nonyloxy and decyloxy), cycloalkyloxy, including cyclopentyloxy, cyclohexyloxy and cycloheptyloxy, aryloxy including phenoxy and phenoxy substituted with halo, alkyl, or alkoxy, haloalkyl which means straight or branched chain alkyl having 1 to 8 carbon atoms which are substituted by at least one halogen, and includes, for example, chloromethyl, bromomethyl, fluoromethyl, iodomethyl, 2-chloroethyl, 2-bromoethyl, 2-fluoroethyl, 3-chloropropyl, 3-bromopropyl, 3-fluoropropyl, 4-chlorobutyl, 4-fluorobutyl, dichloromethyl, dibromomethyl, difluoromethyl, diiodomethyl, 2,2-dichloroethyl, 2,2-diibromoethyl, 2,2-difluoroethyl, 3,3-dichloropropyl, 3,3-difluoropropyl, 4,4-dichlorobutyl, 4,4-difluorobutyl, trichloromethyl, trifluoromethyl, 2,2,2-trifluoroethyl, 2,3,3-trifluoropropyl, 1,1,2,2-tetrafluoroethyl, or 2,2,3,3-tetrafluoropropyl;
"alkylthio" means a divalent sulfur with alkyl occupying one of the valencies and includes the groups methylthio, ethylthio, propylthio, butylthio, pentylthio, hexylthio, or ocrylthio;
"heteroaryl" means 5 to 10 membered mono- or fused-hetero-aromatic ring which has at least one hetero atom and includes those selected from nitrogen, oxygen and sulfur, and includes, for example, 2-furyl, 3-furyl, 2-thienyl, 3-thienyl, 2-pyridyl, 3-pyridyl, 4-pyridyl, pyrazolyl; imidazolyl, pyrimidinyl, pyridazinyl, pyrazinyl, benzimidazolyl, quinolyl, oxazolyl, thiazolyl, or indolyl;
"substituted heteroaryl" means 5 to 10 membered mono- or fused-heteroaromatic ring which has in the ring at least one hetero atom selected from nitrogen, oxygen and sulfur, and which ring is substituted by at least one substituent selected from halogen, amino, nitro, hydroxy, alkyl, alkoxy and haloalkyl on the above-mentioned heteroaromatic nucleus;
"alkanoyl" means alkanoyl having 2 to 18 carbon atoms and includes, for example, acetyl, propionyl, butyryl, isobutyryl, pivaloyl, valeryl, hexanoyl, octanoyl, lauroyl, or stearoyl;
"aroyl" means benzoyl or naphthoyl;
"substituted aroyl" means benzoyl or naphthoyl substituted by at least one substituent including those selected from halogen, amino, nitro, hydroxy, alkyl, alkoxy and haloalkyl on the benzene or naphthalene ring;
"heteroarylcarbonyl" means that the heteroaryl moiety is 5 to 10 membered mono-or fused- heteroaromatic ring having at least one heteroatom selected from nitrogen, oxygen and sulfur as mentioned above, and includes, for example, furoyl, nicotinoyl, isonicotinoyl, pyrazolylcarbonyl, imidazolylcarbonyl, pyrimidinylcarbonyl, or benzimidazolylcarbonyl ;
"substituted heteroarylcarbonyl" means the above-mentioned heteroarylcarbonyl which is substituted by at least one substituent selected from halogen, amino, nitro, hydroxy, alkoxy and haloalkyl on the heteroaryl nucleus; and includes, for example, 2-oxo-1,3-dioxolan-4-ylmethyl, or 2-oxo-1,3-dioxan-5-yl;
"vinyl" means an unsaturated substituent having at least one unsaturated double bond and having the formula CH₂=CH-;
"substituted vinyl" means the above vinyl substituent having at least one of the protons on the terminal carbon atom replaced with alkyl, cycloalkyl, cycloalkylalkyl, aryl, substituted aryl, heteroaryl or substituted heteroaryl;
"hydrocarbyl" means a univalent hydrocarbon group containing up to 24 carbon atoms (i.e., a group containing only carbon and hydrogen atoms) and that is devoid of olefinic and acetylenic unsaturation, and includes alkyl, cycloalkyl, alkyl-substituted cycloalkyl, cycloalkyl-substituted cycloalkyl, cycloalkylalkyl, aryl, alkyl-substituted aryl, cycloalkyl-substituted aryl, aralkyl, alkyl-substituted aralkyl, and cycloalkyl-substituted aralkyl; and
"functionally-substituted hydrocarbyl groups means a hydrocarbyl group that is substituted by one or more functional groups selected from halogen atoms, amino, nitro, hydroxy, hydrocarbyloxy (including alkoxy, cycloalkyloxy, and aryloxy), hydrocarbylthio (including alkylthio, cycloalkylthio, and arylthio), heteroaryl, substituted heteroaryl, alkanoyl, aroyl, substituted aroyl, heteroarylcarbonyl, and substituted heteroarylcarbonyl;
"arylation" means a reaction in which at least one aryl halide, or at least one substituted aryl halide or a combination of at least one aryl halide and at least one substituted aryl halide is used as a reactant in the reaction with at least one olefinic compound;
"liquid" means that the material referred to exists in the liquid state of aggregation at 20°C (and preferably at temperatures below 20°C).

### FURTHER DETAILED DESCRIPTION

### Arylation Reaction

Palladium-catalyzed arylations of olefins with aryl halides are well known and reported in the literature. See for example, C.B. Ziegler, Jr., and R.F. Heck, *J. Org. Chem.,* **1978,** 43, 2941 and references cited therein, and U.S. Pat. No. 5,536,870 to T-C Wu. In the practice of this invention the arylation reaction is used for preparing an olefinic compound of the formula 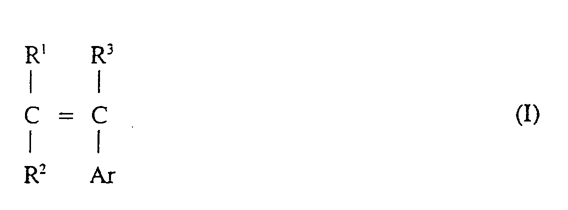 where Ar is aryl, substituted aryl, heteroaryl, substituted heteroaryl, aralkyl (especially benzyl), or substituted aralkyl (especially substituted benzyl), and R¹, R², and R³ are the same or different and are selected from hydrogen atoms, hydrocarbyl groups, functionally-substituted hydrocarbyl groups, and halogen atoms. This is accomplished by reacting at least one aryl halide and/or substituted aryl halide of the formula

Ar-X (II)

where Ar is as defined above and X is a halogen atom of atomic number greater than 9, a diazonium group or triflate or other leaving group; with at least one olefinic compound of the formula 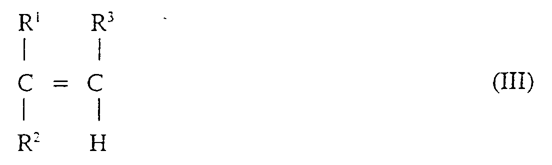 where R¹, R², and R³ are as previously defined. The substituted aryl group of the substituted aryl halide is preferably phenyl substituted with alkyl, naphthyl substituted with alkoxy, phenyl substituted with aryloxy or substituted aryloxy (especially phenoxy), aryl substituted with fluoro, or phenyl substituted with aroyl, and the halogen atom of the substituted aryl halide is preferably a bromine atom. Examples of substituted aryl halides include compounds wherein the substituted aryl group is an isobutylphenyl group, a methoxynaphthyl group, a phenoxyphenyl group, a fluorobiphenylyl group, a benzoylphenyl group, and where the halogen atom is a chlorine, an iodine, or most preferably, a bromine atom.

The preferred olefinic compounds of Formula (III) are those in which R¹, R², and R³ are hydrogen atoms, C₁ to C₆ alkyl, substituted or unsubstituted phenyl, and/or trifluoromethyl. Examples include compounds of Formula (III) wherein R¹, R², and R³ are hydrogen atoms, methyl, and/or trifluoromethyl. Olefins in which R³ is a hydrogen atom are more preferred, and vinyl olefins in which R¹ is a hydrogen atom or a C₁ to C₆ alkyl group, and R² and R³ are hydrogen atoms especially preferred. Ethylene is the most preferred olefinic reactant.

The reaction is conducted in a liquid medium formed from (i) one or more liquid polar organic solvent/diluents, and (ii) one or more secondary or tertiary amines that (1) boil(s) below the boiling temperature of the solvent/diluent if only one solvent/diluent is used in forming the medium or (2) that boil(s) below the boiling temperature of at least one, but not necessarily all, of the polar solvent/diluents used in forming the medium if more than one solvent/diluent is used in forming the medium. The solvent/diluent should have at least a measurable polarity at a temperature in the range of 20 to 25°C, and yet be free of functionality that would prevent or materially impair, inhibit or otherwise materially interfere with the arylation reaction. Examples include tetrahydrofuran, 1,4-dioxane; diglyme, triglyme, acetonitrile, propionitrile, benzonitrile, N,N-dimethylformamide, N,N-dimethylacetamide, dimethylsulfoxide, nitrobenzene, sulfolane, acetone, butanone and cyclohexanone. Preferred solvent/diluents are one or more aprotic solvents each having a dielectric constant of at least 10 (especially 10 to 30) at a temperature in the range of 20 to 25°C. From the cost-effectiveness standpoint, hydrocarbyl ketones with 4 or more carbon atoms in the molecule (e.g., 4 to 8) are especially preferable. Examples include diethyl ketone, methyl isobutyl ketone, 2-pentanone, 2-hexanone, 2-heptanone, 3-heptanone, 4-heptanone, and like liquid ketones, as well as mixtures of two or more such ketones. Most preferred is diethyl ketone (3-pentanone). The arylation reaction inherently tends to be an exothermic reaction, and the use of diluents having a dielectric constant in the range of 10 to 30 (as measured at 20 to 25°C), such as a ketone meeting this qualification provides a readily controllable reaction.

The secondary or tertiary amines are used as hydrogen halide acceptors and thus preferably are used in at least a stoichiometric amount relative to the aryl halide and/or substituted aryl halide being used. However it is possible, though less desirable, to use less than a stoichiometric amount of amine, by allowing the reaction with less than a stoichiometric amount of amine to proceed only part way, and by recycling the reaction mixture for further reaction in the presence of additional amine added thereto.

Use can be made of any liquid secondary or tertiary amine that is free of functionality that would prevent or materially impair, inhibit or otherwise materially interfere with the arylation reaction, that boils below the boiling temperature of the polar solvent/diluent used when only one is used in forming the liquid medium for the reaction, or that boils below at least one of a plurality of polar solvent/diluents used in at least a substantial amount (e.g., at least 20 or 30% of the total volume of the solvent/diluents), when more than one is used in forming the liquid medium for the reaction, and that has sufficient basicity to serve as a hydrogen halide acceptor for the HCl, HBr and/or HI, formed in the arylation reaction. Preferred are liquid tertiary amines. The amines may be polyamines such as for example, N,N,N',N'-tetramethylethylenediamine (b.p. ca. 120-122°C), but in most cases monoamines are preferable. Among useful liquid amines having suitably low boiling points are diethylamine (bp 55°C), N,N-dimethylethylamine (bp 36-38°C), N,N-diethylmethylamine (bp 63-65°C), diisopropylamine (bp 84°C). triethylamine (bp ca. 89°C), dipropylamine (bp ca. 105-110°C), and di-sec-butylamine (bp ca. 135°C). Triethylamine is a particularly preferred amine.

Liquid media formed from diethyl ketone and acetonitrile (e.g. in a weight ratio in the range of 1:9 to 4:1, and more preferably in the range of 1:3 to 3:1) plus triethylamine, or from diethyl ketone and N,N-dimethylformamide (e.g., in a weight ratio in the range of 1:9 to 9:1) plus triethylamine are typical desirable liquid media for use in this invention.

Liquid media formed from diethyl ketone and triethylamine or from methyl isobutyl ketone and triethylamine are particularly preferred.

In the practice of this invention, the reaction is typically conducted in the presence of a catalytically effective amount of a catalyst system formed from (a) palladium and/or at least one compound of palladium in which the palladium has a valence of zero, 1 or 2, and (b) a tertiary phosphine ligand of the formula

R⁴R⁵R⁶P (IV)

where R⁴, R⁵, and R⁶ are the same or different and are selected from alkyl, aryl, substituted aryl, heteroaryl, substituted heteroaryl, aralkyl, substituted aralkyl, cycloalkyl, and substituted cycloalkyl, at least one of R⁴, R⁵, and R⁶ being aryl or substituted aryl. Preferably at least one of R⁴, R⁵, and R⁶ is aryl or substituted aryl and at least one of R⁴, R⁵, and R⁶ is cycloalkyl or substituted cycloalkyl.

The use of salts of palladium in forming the catalysts is preferable because catalyst compositions formed from palladium salts appear to have greater activity than those made from palladium metal itself. Of the salts, palladium(II) salts such as the Pd(II) halides (chloride, bromide, iodide) and Pd(II) carboxylates (e.g., acetate, propionate) are most preferred.

A highly preferred type of tertiary phosphine (sometimes referred to herein as "ligand ") used is one or more tertiary phosphine ligands of the formula 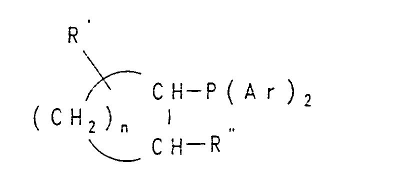 where R' and R" are the same or different and are individually hydrogen, alkyl, aryl or substituted aryl, Ar is phenyl, naphthyl, substituted phenyl or substituted naphthyl and n is an integer from 3 to 6. Preferably, R' and R" are the same or different and are C, to C₆ alkyl, Ar is phenyl or naphthyl and n is 3 or 4. Most preferably, R' is methyl or ethyl, R" is C₃ to C₆ branched alkyl, Ar is phenyl and n is 4. Especially preferred as the phosphine ligand is neomenthyldiphenylphosphine.

Active catalytic species are preferably formed *in situ* by the addition to the reaction mixture of the foregoing individual components. However the catalyst can be preformed externally to the reaction mixture and charged to the reactor as a preformed catalyst composition.

Desirably, a small reaction-accelerating amount of water is included or present in the reaction mixture, as described in commonly-owned U.S. application Serial No. 08/780,310, filed January 8, 1997. This amount is typically in the range of 0.5 to 5 wt% of the total weight of the entire reaction mixture. Within the range of 0.5 to 5 weight percent water there is often an optimum amount of water which gives the highest or peak reaction rate which falls off if more or less water is used. This optimum amount of water may vary depending upon the identity and proportions of the ingredients used in forming the reaction mixture. Thus in any given situation it may be desirable to perform a few preliminary experiments with the particular reaction to be conducted, wherein the amount of water is varied within the range of 0.5 to 5 wt % to locate the optimum rate-enhancing amount of water in the mixture. Preferably, the amount of water used will be insufficient to form a second liquid phase (i.e., a separate water layer) in a mixture consisting of (i) the amount of the liquid organic solvent/diluent(s) selected for use, (ii) the selected amount of the liquid secondary and/or tertiary amine(s) selected for use. and (iii) the selected amount of water, when such mixture is agitated for 10 minutes at 25°C and allowed to stand for 15 minutes at the same temperature. Thus when conducting the process on a large scale with recycle of solvent(s) and amine, the amount of water carried over from product workup should be monitored and/or controlled such that the water content of the reaction mixture remains at or below 5 wt% of the total weight thereof. Conversely if the amount of recycled water is insufficient to maintain the desired water content in the reaction mixture, additional water should be added to bring the water content up to the desired amount within the foregoing range. Preferably the arylation reaction mixtures have a water content in the range of 1 to 3.5 weight percent.

In conducting an operation wherein a mixture of (i) liquid organic solvent/diluents), (ii) secondary and/or tertiary amine(s), and (iii) water that does not separate into a two-phase system is used, the liquid mixture of these components may nonetheless be hazy or cloudy, but a distinct coalesced second liquid phase does not and should not exist as a separate layer in such liquid mixture.

The arylation reaction is performed under conditions such that olefinic compound of Formula (1) above is formed. Such conditions usually require an equimolar ratio of olefinic compound (Formula (III) above) to aryl halide and/or substituted aryl halide (Formula (II) above), although an excess of olefinic compound is preferred. The palladium catalyst and the phosphine ligand are typically used at a ratio of 1 mole of organic halide to 0.0005 mole of palladium or palladium compound. The ligand is present in the same or higher molar proportion as the palladium or palladium compound. It should be noted that levels of (a) palladium or palladium compound, and (b) ligand can be substantially higher (up to 10 times). When relatively inactive species of olefinic compound or aryl halide and/or substituted aryl halide are employed, for example, highly substituted olefins and/or substituted aryl halides bearing strongly electron donating substituents, these higher amounts of catalyst and ligand may be required. Thus the mole ratio of aryl halide and/or substituted aryl halide:Pd:ligand used will generally be a suitable ratio within the range of 200-20,000:1:1-20, respectively.

Temperatures of reaction are quite modest, varying from 25°C to 200°C (preferably 60°C to 150°C) with pressures (for the gaseous vinyl compounds) being from atmospheric up to 3000 psi (preferably 300 to 1000 psi). With the preferred catalyst systems and liquid media referred to above, reaction times are unusually short, typically giving complete reaction in the range of 1 to 24 hours, typically in the range of 2 to 6 hours. Higher temperatures and lower pressures tend to cause increased by-product formation.

The preferred and the optimum conditions will depend to some extent upon the identity of the particular ingredients being used. Thus, for example, when forming 6-methoxy-2-vinylnaphthalene (MVN) from 2-bromo-6-methoxynaphthalene (BMN) using ethylene as the olefinic reactant, a palladium (II) salt such as PdCl₂ and neomenthyldiphenylphosphine (NMDP) as catalyst or catalyst precursors, a C₄-C₈ ketone especially diethyl ketone and a C₄-C₉ trialkyl amine especially triethylamine as the liquid medium and a reaction accelerating amount of water, the BMN:Pd:NMDP mole ratio is preferably in the range of 1000:3000:1:2-10, respectively, the mole ratio of amine:BMN can be in the range of 0.1-2:1 and preferably is in the range of 1-2:1 respectively, the mole ratio of ketone:amine is preferably in the range of 1.0-4.0:1 respectively, the weight of water based on the total weight of BMN + ketone + amine + Pd catalyst ingredient + tertiary phosphine ligand + water is preferably in the range 1 to 3.5 wt%, the reaction temperature is typically in the range of 60 to 150°C and preferably in the range of 80 to 110°C, and the pressure of the ethylene used is preferably in the range of 400 to 1000 psig. Under these conditions, reaction is complete within 1 to 24 hours, and oftentimes within 2 to 6 hours, with conversions and yields of MVN (both based on BMN used) of 70% to 99%, such as, for example, 95% conversion and 85% yield. It is to be clearly understood that the foregoing conditions given in this paragraph are, as stated, *preferred* conditions for carrying out the specified reaction. On the basis of the information presented in this disclosure, one skilled in the art could readily operate outside of the ranges given in this paragraph, and still achieve good performance in accordance with this invention. Thus this invention is not limited to use of the conditions given in this paragraph, and it is within the scope of this invention when performing the specified reaction to depart from any one or more of such ranges, whenever deemed necessary or desirable in any given situation.

For best results, the overall arylation reaction mixture is essentially solids-free when at reaction temperatures, except for some precipitation of palladium and formation of some solid co-products such as amine-hydrohalide salt and products formed by interaction of the aryl halide and/or substituted aryl halide (e.g., BMN) with the vinylated product (e.g., MVN), and/or by dimerization of such vinylated product that may occur as the reaction proceeds. Since the reaction tends to be exothermic, it is desirable to utilize reactors equipped with internal cooling coils, cooling jackets or other highly effective cooling means to ensure suitable temperature control.

A few examples of desirable laboratory reaction parameters in the reaction of BMN with ethylene using PdCl₂ and NMDP at 95°C and 420 psig ethylene are as follows:
a) NMDP:Pd mole ratios in the 5:1-6:1 range give relatively fast reaction rates.
b) BMN:Pd:NMDP mole ratios of 2000:1:6, 2500:1:5 and 3000:1:10 give high conversions and good yields; ratios of 3000:1:6 and 3500:1:5 are operable but give lower conversions.
c) As agitator speeds increase from 300 to 1500 rpm, reaction times to completion decrease by almost two hours.
d) At a BMN:Pd:NMDP mole ratio of 2000:1:6, ethylene pressures ranging from 190 psig to 955 psig at 95°C give good results. Thus at 190 psig the yield of MVN was 86%, and at 900 psig the yield was 96%. At the higher pressures of the range, reaction times were shorter and the amount of solid by-products formed was less.
e) At a BMN:Pd:NMDP mole ratio of 2000:1:6, MVN yields are higher and the amount of solid by-products formed is lower, when using BMN concentrations at the lower end of the range of 20 to 35 wt% than at the higher end of the range.
f) Reactions at a BMN:Pd:NMDP mole ratio of 2000:1:6 using 1.6 wt% water as reaction accelerator proceed at a higher reaction rate at 95°C than at 85°C.
g) Maximum rate of reaction is achieved at 3 wt% water when operating at 95°C, 420 psig ethylene, BMN:Pd:NMDP mole ratio of 2000:1:6, at 30 wt% BMN concentration. The rate is 150% of the rate when no added water is present. Under these particular conditions, water levels greater than 4% caused the reaction to stop at less than complete conversion.
h) Use of recycled DEK solvent in four successive runs was successful: no new impurities were found in the MVN product solutions after four recycles. Addition of makeup water when needed to maintain the desired level of water in the reaction mixture is desirable in order to achieve the beneficial reaction accelerating effect of the water from run to run.

Therefore, in producing 6-methoxy-2-vinylnaphthalene (MVN) from 2-bromo-6-methoxynaphthalene (BMN) by reaction with ethylene, using a palladium (II) salt such as PdCl₂ and neomenthyldiphenylphosphine (NMDP) as catalyst or catalyst precursors, the preferred reaction medium is a mixture comprising a C₄-C₈ ketone (especially diethyl ketone) and a C₄-C₉ trialkyl amine (especially triethylamine). This reaction medium preferably contains a reaction accelerating amount of water in the range of 1 to 3.5 weight percent of the total weight of the reaction mixture. The BMN:Pd:NMDP mole ratio is preferably in the range of 1000:3000:1:2-10, respectively, (e.g., a BMN:Pd:NMP mole ratio of 2000:1:6), the mole ratio of amine:BMN is preferably in the range of 1-2:1 respectively, the mole ratio of ketone:amine is preferably in the range of 1.0-4.0:1 respectively, the reaction temperature is preferably in the range of 80 to 110°C (e.g., 95°C), and the pressure of the ethylene used is preferably in the range of 400 to 1000 psig (e.g., 420 psig).

### Workup of Arylation Product

The arylation reaction produces a reaction mixture comprising olefinically-substituted aromatic compound, amine-hydrohalide and one or more of the polar organic solvents. Pursuant to one of the preferred embodiments of this invention, a concentrated aqueous solution of inorganic base such as K₂CO₃, NaHCO₃, having a base strength greater than that of the amine(s) of the amine hydrohalide, and more preferably a concentrated aqueous alkali metal hydroxide solution, is mixed with at least a portion (preferably, all) of the reaction mixture to convert the amine-hydrohalide therein to free amine and inorganic halide salt such as alkali metal halide, and to form (i) an aqueous phase containing dissolved halide salt, and (ii) an organic phase comprising olefinically-substituted aromatic compound, amine, and one or more of the polar organic solvents. Although well known to those skilled in the art, it is deemed necessary, or at least prudent, to point out that because the conversion of the amine-hydrohalide to free amine and, say, "alkali metal halide" is conducted in the presence of water, the "alkali metal halide", or at least a substantial proportion thereof, exists in ionic form while dissolved in the water. Thus according to known chemical principles, the water contains alkali metal cations and halide anions. However chemists would commonly refer to this as forming alkali metal halide because upon removal of water, alkali metal halide would indeed exist as such. Thus when referring in the specification and claims hereof to convening the amine-hydrohalide to free amine and halide salt such as alkali metal halide, it is to be understood that this means that the resulting mixture contains the liberated amine and the halide salt in whatever chemical forms they exist in the environment and under the conditions used.

The concentrated alkali metal hydroxide solution may be formed by dissolving alkali metal oxide or hydroxide, or both. in water. The preferred alkali metal oxides and/or hydroxides are those of sodium or potassium, or mixtures thereof. These are plentiful and less expensive than the lithium, rubidium and cesium oxides and hydroxides, which could, however, be used. If desired, the sodium hydroxide or potassium hydroxide solution may be formed from small or even trace amounts of one or more of these other more expensive alkali metal oxides and/or hydroxides together with large amounts of the sodium and/or potassium oxides and/or hydroxides. Again it is to be noted that in the aqueous solution, the alkali metal hydroxide is ionized so that the solution contains, according to well established chemical principles, alkali metal cations and hydroxyl anions. Therefore, reference in the specification and claims hereof to alkali metal hydroxide solution means that the alkali metal hydroxide is in whatever chemical form it exists while in a concentrated aqueous solution.

Whether conducted in stages or all at once, ultimately at least a stoichiometric amount of the inorganic base should be, and in most cases is, employed relative to the amount of amine-hydrohalide present in the reaction mixture.

As to the concentration of these inorganic base solutions, it is desirable to use solutions that contain the equivalent of at least 10 weight percent of the base, such as alkali metal hydroxide, being used. Saturated aqueous alkali metal hydroxide solutions can be used, but typically the concentration will be at least slightly less than this. Preferred aqueous solutions contain the equivalent of 20 to 50 wt% of sodium hydroxide or of potassium hydroxide, or of both. Particularly preferred aqueous solutions contain the equivalent of 23 to 27 wt% of sodium hydroxide and/or potassium hydroxide. Most preferred is 25 wt% sodium hydroxide aqueous solution.

Preferably the aqueous solution of inorganic base such as alkali metal hydroxide is used in an amount that produces an alkali metal halide solution containing the equivalent of at least 30 wt% of sodium bromide, and more preferably the equivalent of at least 40 to 50 wt% of sodium bromide, as this makes the ensuing phase separation easier if the aqueous phase has the higher densities of such concentrated solutions. In addition, less of the organic solvent/diluent(s) and amine(s) are soluble in the aqueous phases having such higher metal halide concentrations, and thus solvent losses are thereby reduced.

The conditions for the mixing of the inorganic base solution such as alkali metal hydroxide solution with the arylation reaction mixture are not critical. All that is required is to ensure that these materials are sufficiently well mixed so that intimate contact is established between these materials. Temperatures will typically be in the range of 40 to 70°C, but other temperatures may be used. Agitation periods in the range of 5 to 15 minutes will normally suffice, but longer periods of up to 30 minutes or more (e.g., one hour or more) can be used, if desired.

After mixing, the resulting mixture is allowed or caused to separate into the organic and aqueous phases, usually by allowing the mixture to stand in a quiescent state. Standing periods of one hour or less are usually sufficient. In fact, when treating an arylation reaction mixture with sufficiently concentrated sodium hydroxide solution to produce an aqueous phase containing 40-45 wt% of sodium bromide, the phases separate quickly, e.g., in as little as 15 minutes. Moreover the phase interface is distinct and easy to detect since oligomeric coproducts tend to float on top of such a concentrated aqueous phase. Then the phases are separated from each other, for example by decantation or, more usually, by draining off the lower aqueous layer.

Next, substantially all of the amine is distilled from the remainder of the organic phase under low temperature and pressure conditions that suppress thermal oligomerization of the olefinically-substituted aromatic compound contained in the residual liquid phase. This distillation can be performed at any suitable reduced pressure such as, for example, in the range of 50 to 600 mm Hg, and preferably at pressures in the range of 200 to 350 mm Hg. Residual amine if present in excessive amounts in the remainder of the organic phase after distillation can have adverse effects upon the ensuing carboxylation reaction. For example, excessive amounts of such residual amine can cause the carboxylation reaction to stop prematurely with consequent loss of conversions and yields. The amount of such residual amine that can be tolerated in the remainder of the organic phase after distillation may vary depending upon such factors as the makeup of the organic phase, the identity of olefinically-substituted aromatic compound contained therein, and the conditions to be used in the carboxylation reaction. Thus in any given situation it may be desirable to perform a few preliminary experiments to determine the amount of amine that can be tolerated without significant adverse effects. Thus sufficient amine is removed such that residual amine, if any, remaining in the remainder of the organic phase does not cause (a) more than a 5% reduction in conversion of olefinically-substituted aromatic compound contained in the remainder of such organic phase, and (b) more than a 5% loss of yield of carboxylated product in the ensuing carboxylation as compared to an identical carboxylation of another portion of the same original organic phase from which the amine has been rigorously removed to the extent possible without significantly reducing the olefinically-substituted aromatic compound content of the organic phase. Preferably the amount of residual amine, if any, remaining in the remainder of the organic phase is sufficiently small so that (a) no more than a 1% reduction in conversion of olefinically-substituted aromatic compound contained in the remainder of such organic phase, and (b) no more than a 1% loss of yield of carboxylated product in the ensuing carboxylation will occur as compared to an identical carboxylation of another portion of the same original organic phase from which the amine has been rigorously removed to the extent possible without significantly reducing the olefinically-substituted aromatic compound content of the organic phase. To ensure no material adverse effects of amine on the carboxylation reaction, residual amounts of amine are preferably maintained below one (1) percent by weight of the distilland remaining after the distillation of amine therefrom.

Preferably, liquid organic makeup solvent is mixed with the liquid mixture during or after the distillation of the amine whereby the liquid mixture for carboxylation further comprises at least a portion (preferably, all) of the distilland and the makeup solvent. While various solvents may be used. the makeup solvent preferably comprises at least one ether, preferably a liquid cyclic monoether such as tetrahydrofuran, methyl-tetrahydrofuran, or tetrahydropyran, or a cyclic diether such as 1,3-dioxolane, or 1,4-dioxane, or a mixture of such materials with or without one or more acyclic ethers such as diethyl ether, methyl tert-butyl ether, or the like. The most preferred makeup solvent is tetrahydrofuran as this material appears to exert a rate enhancing effect upon the carboxylation reaction. It is expected that at least some alkyl-substituted tetrahydrofurans may also behave in this manner.

When a mixture of acetonitrile and a liquid ketone having a boiling point above the amine, such as diethyl ketone and/or methylisobutyl ketone, is used as the solvent or diluent in the arylation reaction, minor variants in the workup procedure are preferably employed. In one such procedure, (1) the acetonitrile is distilled from the arylation reaction mixture, (2) the concentrated aqueous solution of inorganic base is mixed with the residual reaction product to form the aqueous and organic phases (as above), (3) the phases are separated, and (4) the amine is distilled from the organic phase. Then makeup solvent (e.g., tetrahydrofuran) is added to the organic phase, and the resultant organic phase is then utilized in the carboxylation reaction described more fully hereinafter. Another such procedure involves (1) mixing the concentrated aqueous solution of inorganic base with the arylation reaction mixture, (2) separating the phases, and (3) distilling the acetonitrile and the amine from the separated organic phase. Then the makeup solvent is added to the organic phase, and the resultant organic phase is then utilized in the carboxylation reaction.

Another process for effecting workup of the arylation reaction product involves using a dilute aqueous washing procedure. In this embodiment the procedure comprises mixing with at least a portion of the arylation reaction product composition a dilute aqueous acid to thereby form (i) an organic phase containing the arylolefin or substituted arylolefin, and (ii) an acidic aqueous phase containing dissolved amine hydrohalide, and separating at least a portion of these phases from each other. The dilute aqueous acid is preferably dilute aqueous hydrochloric acid, e.g., in the range of 1 to 20 wt% aqueous HCl. The amount used should be sufficient to form an acidic aqueous phase containing substantially all of the amine-hydrohalide, which can readily be separated from the organic phase comprising the polar solvent(s) and the arylolefin or substituted arylolefin. At least a portion of the separated organic phase is then suitable as feed to a palladium-catalyzed carboxylation to form arylcarboxylic acid or ester or substituted arylcarboxylic acid or ester in accordance with conditions and procedures described hereinafter. Before conducting the carboxylation reaction, an ethereal solvent such as a cyclic ether solvent (tetrahydrofuran, methyl-tetrahydrofuran, or 1,4-dioxane), can be added to the separated organic phase to enhance the ensuing carboxylation reaction. To accommodate the added ethereal solvent, the separated organic phase may be subjected to a stripping or distillation step to remove some of the polar solvent(s) from the separated organic phase, before adding the ethereal solvent. The stripped polar solvent may be used as recycle solvent in the arylation process.

It is also desirable to recover the secondary and/or tertiary amine from the separated aqueous phase. This is accomplished by mixing together at least a portion of the separated aqueous phase and a strong inorganic base to form free amine and an aqueous solution of inorganic halide. Suitable strong bases include NaOH, KOH, NH₄OH, Na₂O, K₂O, Ca(OH)₂, Na₂CO₃, K₂CO₃, CaO, and other inorganic bases of comparable base strength. This results in the formation of an aqueous phase and an organic phase consisting essentially of the free amine(s). Separation of these phases provides the amine for use as recycle. The amine can be purified by distillation, if necessary.

### Carboxylation

In this operation the olefinically-substituted aromatic compound is converted into a compound of the formula 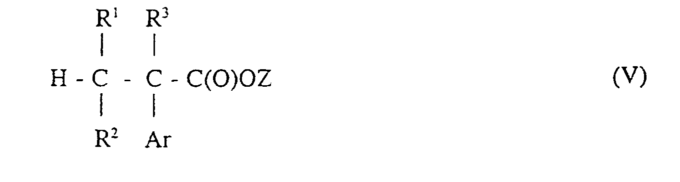 where Ar, R¹, R², and R³ are as previously defined, and Z is a hydrogen atom, an alkali metal atom (preferably Na or K). a hydrocarbyl group (preferably C₁-C₆ alkyl), or a functionally-substituted hydrocarbyl group. The procedures and conditions for effecting the carboxylation leading to the formation of compounds of Formula (V) are described below. By suitable modifications of or additions to such procedures, compounds of Formula (V) can be produced in which Z can be any of a wide variety of other groups, non-limiting exemplifications of which include ammonium, quaternary ammonium, one-half equivalent of a divalent metal atom, one-third equivalent of a trivalent metal cation, and so on.

The catalytic carboxylation of the compound of Formula (I) is effected with carbon monoxide and water and/or alcohol, and is conducted, at a temperature between 25°C and 200°C, preferably 25°-120°C, and most preferably 25°-100°C. Higher temperatures can also be used. The best yields are obtained when the temperature is maintained at a relatively low level throughout the reaction.

The partial pressure of carbon monoxide in the reaction vessel is at least 1 atmosphere (0 psig) at ambient temperature (or the temperature at which the vessel is charged). Any higher pressures of carbon monoxide can be used up to the pressure limits of the reaction apparatus. A pressure up to 3000 psig is convenient in the process. More preferred is a pressure from 0 to 3000 psig at the reaction temperature and most preferred is a pressure from 0 to 1000 psig. It should be noted that the presence of oxygen is undesirable in the hydrocarboxylation reaction of this invention. Hence, an atmosphere of 100% carbon monoxide is most preferred to carry out this process. Various inert gases can, however, be incorporated in the reaction mass (nitrogen, or argon), the only criterion being that the process should not be slowed to the point of requiring exceptionally long periods to complete the reaction.

As noted above, the carboxylation is conducted in the presence of an appropriate amount of water or aliphatic alcohol. Strictly speaking, when the reaction is conducted in the presence of water it is a hydrocarboxylation reaction, and when conducted in the presence of an alcohol it can be termed a hydrocarbalkoxylation reaction. Consequently, unless otherwise qualified or specified, the term "carboxylation" is used herein in a generic sense to denote both hydrocarboxylation (using water) and hydrocarbalkoxylation (using an alcohol).

In the case of hydrocarboxylation of MVN, at least one (1) mole of water per mole of the MVN should be used, and four moles of water per mole of the MVN is typically employed. It is worth noting that an excessive amount of water can inhibit or even kill the reaction. The effect of large excesses of alcohols in the hydrocarbalkoxylation of MVN has not been studied in detail, but it would appear prudent to avoid use of excessive amounts. Thus amounts in the range of up to 10 wt% in the reaction mixture are suggested. In carboxylation reactions with other compounds of Formula (I), an excess amount of water and/or alcohol may sometimes be used. In such cases, although possibly there may be no real upper limit to the amount of water or alcohol except that imposed by practicality (e.g. the size of the reaction vessel. and the kinetics of the reaction), an amount up to 100 moles, and preferably up to 50, moles per mole of the compounds of Formula (I) may be considered for use in the process, and an amount from 2 to 24 moles of water or alcohol per mole of the such olefinic compound is more preferred. The product of the reaction is a carboxylic acid (where Z in Formula (V) is a hydrogen atom) or carboxylic acid ester (where Z in Formula (V) is alkyl or substituted alkyl).

The present invention embraces the formation of any racemates and individual optical isomers of the compounds of Formula (V) having a chiral carbon atom. For example, when compounds of Formula (V) wherein the acid is 2-(6-methoxy-2-naphthyl)propionic acid, are subjected to resolution as taught in U. S. Patent 4,246,164, the analgesic compound naproxen is produced.

If desired, any alcohol which produces an ester of the carboxylic acid may be used in the practice of this invention. In a preferred embodiment, the C₁ to C₆ aliphatic alcohols are used. Examples of the alcohols to be used in this embodiment include methyl alcohol, ethyl alcohol, n-propyl alcohol, isopropyl alcohol, n-, iso-, sec-, and tert-butyl alcohols, the pentyl alcohols, (isoamyl alcohol, especially to form the (±)-2-(6-methoxy-2-naphthyl)propionic acid ester), or the hexyl alcohols. Methyl alcohol is highly preferred, and ethyl alcohol is most highly preferred. Other alcohols, glycols, or aromatic hydroxy compounds may also be used. In the broadest sense, these alcohols provide a source of alkoxide ions for this reaction. However, any other "source of alkoxide ions" may also be used. The source of such alkoxide ions is from a compound selected from the group consisting of HC(OR₁)₃, (R)₂=C(OR₁)₂, HC(O)OR₁, B(OR₁)₃, Ti(OR₁)₄ and Al(OR₁)₃ where R is hydrogen or individually the same as or different from R₁, and R₁ is alkyl or substituted alkyl.

In some cases, the carboxylation reaction is initiated under neutral conditions, i.e., with no added acid. However, at least in the case of hydrocarboxylation of MVN, the inclusion of aqueous HCl in the reaction mixture is deemed important, if not almost essential for most efficient operation. Thus in a preferred embodiment of this invention, the hydrocarboxylation reaction is initiated in the presence of halide ions which are best provided by use of a halogen acid, especially hydrochloric acid, which preferably is an aqueous acid which may for example have a concentration up to 25 wt%, but preferably has a concentration in the range of 5 to 15 wt%, and more preferably in the range of 7 to 15 wt%. It is especially preferred to use approximately 10 wt% aqueous HCl. Dilute aqueous HCl also provides water for effecting the hydrocarboxylation. Gaseous HCl can be used to generate hydrochloric acid *in situ* when water is present when conducting this reaction. HBr and hydrobromic acid may be used, but these appear less effective based on studies conducted to date. Other acids may be considered for use but to date the most effective material is the aqueous hydrochloric acid. Any suitable proportion of hydrochloric acid may be used, typically a reaction accelerating quantity in the range that provides up to 1 mole of hydrogen ion per mole of compound of Formula I. and preferably a quantity that provides in the range of 0.1 to 0.5 mole of hydrogen ion per mole of the compounds of Formula I. In the case of carboxylation of MVN, the preferred range is an HCl:MVN mole ratio of 0.1 to 0.3, more preferably 0.15 to 0.27, and most preferably 0.18 to 0.22.

The catalytic carboxylation process of this invention is conducted in the presence of a reaction-promoting quantity of (i) palladium and/or at least one palladium compound in which the palladium has a valence of zero, 1 or 2, (most preferably 2) or (ii) a mixture of (a) palladium and/or at least one palladium compound, and (b) at least one copper compound, with (iii) at least one tertiary phosphine of the type described above. When a copper compound is not employed, the palladium and/or one or more compounds of palladium used in forming the catalyst is/are sometimes collectively referred to herein for convenience as "the Pd ingredient", and the combination of palladium and/or one or more compounds of palladium and one or more compounds of copper used in forming the catalyst (when a copper compound is employed) is sometimes collectively referred to herein for convenience as "the Pd-Cu ingredient".

Thus in general the Pd ingredient and the tertiary phosphine ligand are the same type of materials as described above in connection with the arylation reaction. Indeed the same preferred types of materials preferred for use in the arylation reaction are preferred for use in the carboxylation reaction. Fresh catalyst is employed for each such reaction, however. The same species of Pd ingredient and the same species of tertiary phosphine ligand need not be used in these two reactions. Either such component or both of them might differ. Thus, for example, palladium(II) chloride and triphenyl phosphine might be used in the arylation and palladium(II) acetate and tri-o-tolylphosphine might be used in the carboxylation, or vice versa, but in the most preferred case the same species (PdCl₂ and neomenthyldiphenyl-phosphine) are in fact used in both such reactions.

As in the case of the arylation reaction, active catalytic species are preferably formed *in situ* by the addition to the reaction mixture of the individual components. However the catalyst can be preformed externally to the reaction mixture and charged to the reactor as a preformed catalyst composition.

When it is desired to use a copper compound in forming the carboxylation catalyst system, copper complexes such as copper acetylacetonates, copper alkylacetoacetates, or other chelated forms of copper may be used. The preferred copper compounds for this use, however, are salts especially divalent copper salts such as the halides (chloride, bromide, iodide) of copper(II) and the carboxylates of copper(II) such as copper(II) acetate, copper(II) propionate.

In one embodiment, the Pd ingredient and copper compounds are inorganic salts and are added as a preformed complex of, for example, a complex formed from palladium(II) chloride or bromide, copper(II) chloride or bromide and carbon monoxide, or any other similar complex. In a preferred embodiment, active catalytic species are formed *in situ* by the addition to the reaction mixture of the individual components, i.e., either (i) at least one tertiary phosphine and at least one palladium compound such as the inorganic or carboxylate salts of palladium(II), or (ii) at least one tertiary phosphine, at least one copper compound, and at least one palladium compound such as the inorganic or carboxylic salts of palladium(II) and copper(II). These inorganic salts include the chlorides, bromides, nitrates, and sulfates. Organic palladium and/or copper compounds that may be used include complexes and salts such as the carboxylates, e.g., the acetates or propionates. In one preferred embodiment, neomenthyldiphenylphosphine. copper(II) chloride, and palladium(II) chloride are used and are added individually or together, either simultaneously or sequentially. In another preferred embodiment, neomenthyldiphenylphosphine and palladium(II) chloride are used and are added individually or together, either simultaneously or sequentially.

The Pd ingredient or the Pd-Cu ingredient may be supported on carbon, silica, alumina, zeolite, clay and other polymeric materials. but use of a homogeneous catalyst system is definitely preferable.

The amount of the Pd ingredient or of the Pd-Cu ingredient employed is preferably such as to provide from 4 to 8000 moles of the compound of Formula (I) per mole of the Pd ingredient or per total moles of the Pd-Cu ingredient. More preferred is an amount to provide from 40 to 4000 moles (most preferably 20 to 2000 moles) of the compounds of Formula (I) per mole of the Pd ingredient or per total moles of the Pd-Cu ingredient. The process of this invention is conducted in the presence of at least one mole of the tertiary phosphine per mole of the Pd ingredient or per total moles of the Pd-Cu ingredient. More preferably, 1 to 40 moles of tertiary phosphine are used per mole of the Pd ingredient or per total moles of the Pd-Cu ingredient, and most preferably 1 to 20 moles of tertiary phosphine are used per mole of the Pd ingredient or per total moles of the Pd-Cu ingredient.

The presence of a solvent is not always required in the carboxylation reaction, although it is desirable in some circumstances. Those solvents which can be used include one or more of the following: ketones, for example, acetone, methyl ethyl ketone, diethyl ketone, methyl n-propyl ketone, acetophenone, or cyclohexanone; linear, poly and cyclic ethers, for example, diethyl ether, di-n-propyl ether, di-n-butyl ether, ethyl n-propyl ether, glyme (the dimethyl ether of ethylene glycol), diglyme (the dimethyl ether of diethylene glycol), tetrahydrofuran, dioxane, 1,3-dioxolane, and similar compounds; and aromatic hydrocarbons, for example, toluene. ethyl benzene, xylenes, and similar compounds. Alcohols are also suitable as solvents, for example, methanol, ethanol, 1-propanol, 2-propanol isomers of butanol, isomers of pentanol. Esters may also be used, such as ethyl acetate. When an ester or an alcohol is used as solvent, the product is usually the corresponding ester of the carboxylic acid. Most highly preferred are ethers, especially tetrahydrofuran, or mixtures of one or more ethers and one or more ketones, especially mixtures of tetrahydrofuran and diethylketone. When solvents are used, the amount can be up to 100 mL per gram of the compounds of Formula I, but the process is most advantageously conducted in the presence of 1 to 30 mL per gram of the compound of Formula (I).

In those specific embodiments of this invention in which an ester is produced, e.g. ibuprofen alkyl ester, the ester may be conveniently converted to the acid (ibuprofen itself) by conventional methods of hydrolysis. Base hydrolysis can also be employed if desired to produce pharmaceutically acceptable salts wherein the cation is sodium, potassium. calcium, hydrogen carbonate or a quaternary ammonium compound.

### Workup and Recovery of Carboxylation Product

As noted above, the carboxylation reaction forms a reaction product composition comprising arylcarboxylic acid or substituted arylcarboxylic acid (e.g., racemic 2-(6-methoxy-2-naphthyl)propionic acid or 2-(4-isobutylphenyl)propionic acid) or an ester thereof (depending on whether water or an alcohol is used in the carboxylation process), and a liquid medium comprising polar organic solvent (preferably one or more ketones), water and/or alcohol, HCl, and preferably at least one ether (e.g., THF) with a boiling temperature below that of at least one such polar solvent. Also present are catalyst residues and typically some coproducts formed during the reaction.

Pursuant to a preferred embodiment of this invention, the arylcarboxylic acid or substituted arylcarboxylic acid is converted *in situ* into an inorganic salt of such acid by reaction with an aqueous solution of inorganic base (neutralization step). In addition, when the reaction product composition contains (i) at least one low boiling ether (e.g., THF) and/or (ii) at least one low boiling polar solvent, where either or both such low boiling materials boil(s) below the boiling temperature of at least one polar solvent contained in the reaction product mixture, some or all of such low boiling materials are distilled from the reaction product composition (distillation step). If the reactor overheads are susceptible to attack by aqueous HCl, the neutralization step should precede or at least be conducted concurrently with the distillation step. On the other hand, if the reactor overheads are formed from acid-resistant materials of construction, the distillation step can precede and/or follow and/or be conducted concurrently with the neutralization step; the HCl in the mixture will not cause excessive corrosion of the reactor overheads even if the distillation precedes the neutralization. In whatever sequence the neutralization step and the distillation step are conducted, a mixture of residual organic phase and an aqueous phase containing dissolved inorganic salt of the arylcarboxylic acid or substituted arylcarboxylic acid remain in the reactor as a distillation residue (distilland or pot residue). These phases are separated from each other. The aqueous phase is then subjected to a distillation, preferably at or near atmospheric pressure, to remove residual organic impurities such as, for example, THF and DEK. At this point it is desirable to ensure that the residual aqueous phase has a concentration in the range of 10 and 35 wt% of dissolved inorganic salt of the arylcarboxylic acid or substituted arylcarboxylic acid and where necessary, adjusting the concentration of the aqueous phase to 10 and 35 wt% solution by removal or addition of water. The aqueous solution is then washed (extracted) with substantially non-polar liquid organic solvent (preferably a paraffinic solvent, or an aromatic hydrocarbon solvent, such as toluene or xylene), preferably at least twice. The free arylcarboxylic acid or substituted arylcarboxylic acid is then produced by mixing non-oxidizing mineral acid (e.g., sulfuric acid) with the aqueous phase in the presence of substantially non-polar liquid solvent to form (i) an organic phase composed of a solution of arylcarboxylic acid or substituted arylcarboxylic acid in substantially non-polar liquid solvent and (ii) an aqueous phase. After separating these phases from each other, arylcarboxylic acid or substituted arylcarboxylic acid is crystallized from the substantially non-polar liquid solvent.

The aqueous solution of inorganic base used in the above neutralization step is preferably a 10 to 50 wt% solution of NaOH or KOH. However other inorganic bases that can be used include Na₂O, K₂O, Ca(OH)₂, CaO, Na₂CO₃, K₂CO₃, and other inorganic bases of similar basicity. Such solutions are used in an amount at least sufficient to neutralize the arylcarboxylic acid or substituted arylcarboxylic acid and the HCl present in the reaction product composition.

When the carboxylation reaction is conducted using an alcohol so that an ester of the arylcarboxylic acid or substituted arylcarboxylic acid is present in the reaction product composition, it is preferred to saponify the ester *in situ* by mixing a concentrated aqueous solution of a strong inorganic base such as NaOH or KOH with the reaction product composition and applying sufficient heat (e.g., heating to a temperature in the range of up to 80°C) to form the inorganic salt of the arylcarboxylic acid or substituted arylcarboxylic acid. Then the workup procedure for the carboxylation product as described above is carried out.

The low boiling materials recovered in the initial distillation step are preferably recycled for use in the hydrocarboxylation reaction.

Examples of compounds that can be produced by use of the invention include ibuprofen, 2-(4-isobutylphenyl)propionic acid (U.S. Pat. Nos. 3,228,831 and 3,385,886); 2-(3-fluoro-4-biphenylyl)-propionic acid (also known as flurbiprofen) (U.S. Pat. No. 3,755,427): racemic 2-(6-methoxy-2-naphthyl)propionic acid which can be resolved to d-2-(6-methoxy-2-naphthyl)propionic acid (also known as naproxen) (U.S. Pat. No. 3,637,767); α-dl-2-(3-phenoxyphenyl)propionic acid (also known as fenoprofen) (U.S. Pat. No. 3,600,437); and 2-(3-benzoylphenyl)propionic acid (also known as ketoprofen) (U.S. Pat. No. 3,641,127). As described herein, the bromo precursor of each of the above compounds is reacted with an olefinic compound of Formula (III) (most preferably ethylene) in a one-phase organic liquid medium (most preferably a mixture of a liquid ketone, especially diethyl ketone, and a liquid secondary or tertiary amine such as a trialkyl amine, especially triethyl amine), that also preferably contains the above-described reaction accelerating amount of water) in the presence of a palladium catalyst system (as described herein), which is formed from Pd, Pd(I) salt or preferably Pd(II) salt and a tertiary phosphine ligand such as neomenthyldiphenylphosphine. The amine should be selected to avoid beta hydride elimination under reaction conditions and should not react with the olefin or bromo precursor to any appreciable extent. The bromo precursor substitutes on the ethylene to provide the substituted olefin which is then worked up as described above, and then carboxylated (using carbon monoxide and a palladium-phosphine or a palladium-copper-phosphine catalyst system as described herein) to produce the corresponding acid product (if water forms part or all of the solvent system) or the corresponding ester (if an alcohol such as methyl, ethyl or isoamyl alcohol) is used as all or part of the solvent.

Some of the above reactions can be exemplified as follows:

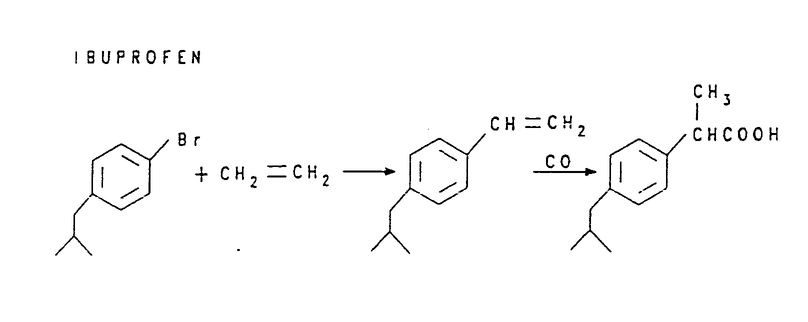

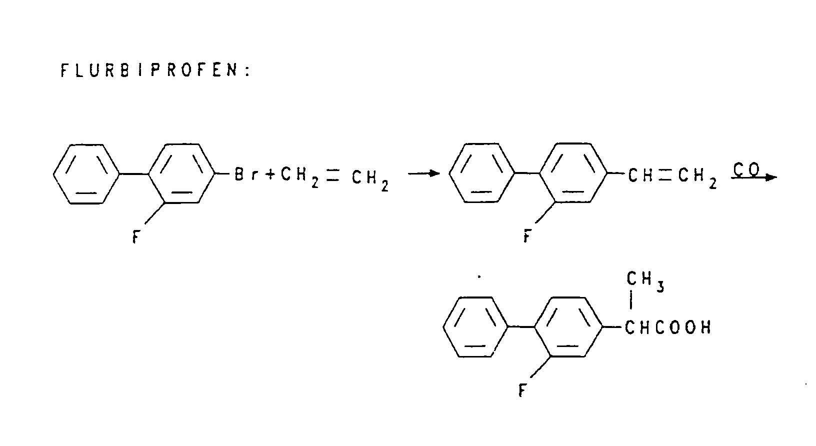

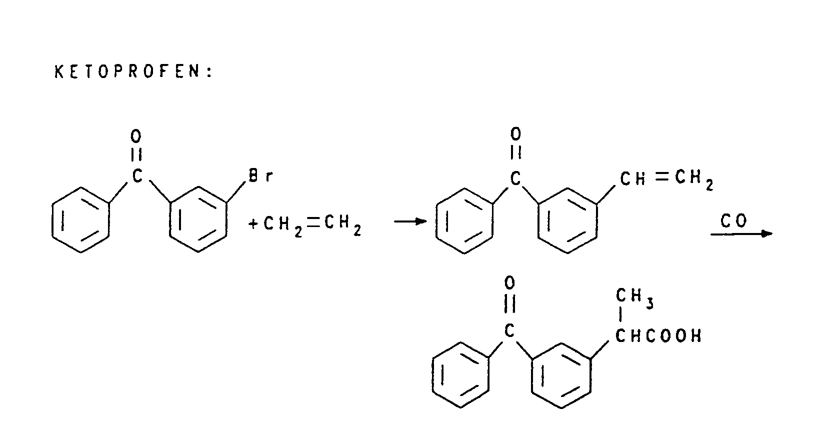

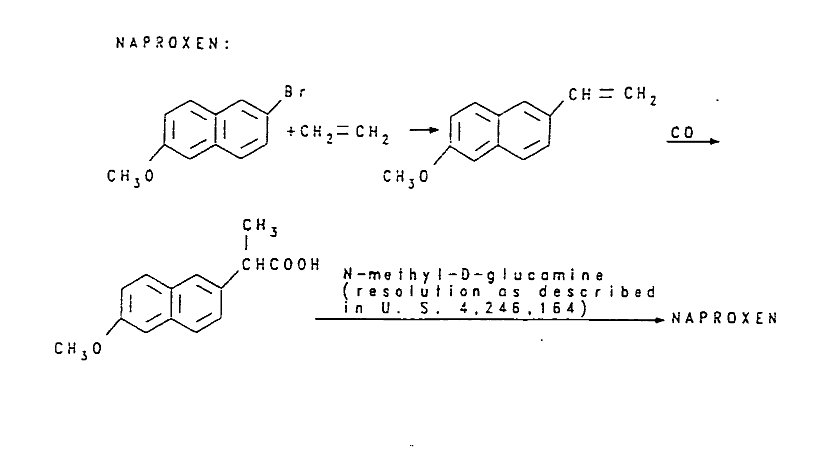

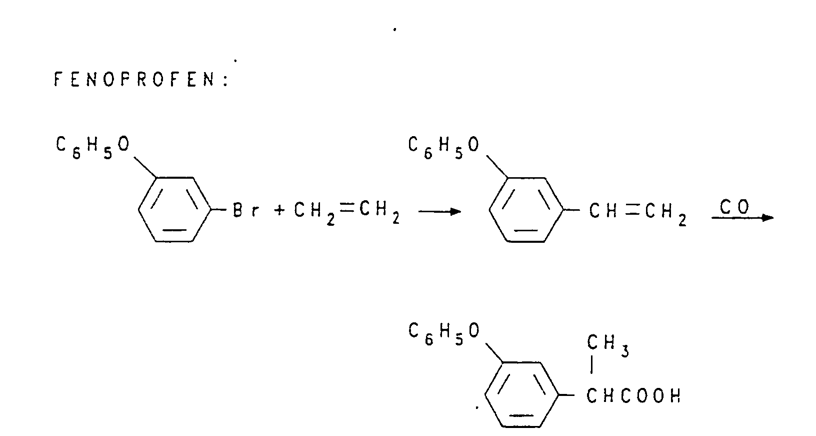

In the above reactions the ethylene pressure should be 50 to 3000 psi (preferably 300 to 1000 psi), the temperature is 30°C to 200°C (preferably 60°C to 150°C). Temperatures and pressures are selected to minimize by-product formation. Palladium is used (i.e., charged to the reactor) preferably in the form of its salts, (e.g., Pd(II) acetate or chloride) along with a tertiary phosphine ligand as described above, with a cycloalkyldi(alkylphenyl)phosphine such as neomenthylditolylphosphine being preferred, and a cycloalhyldiphenyl-phosphine such as neomenthyldiphenylphosphine being particularly preferred.

The bromo precursors are frequently commercially available and/or can be readily prepared by those skilled in the art. For example, Aldrich Chemical Company sells m-bromophenol and m-bromoanisole while Albemarle PPC (Thann, France) sells 2-bromo-6-methoxynaphthalene. The bromo precursors of ibuprofen can be prepared by bromination using standard Friedel-Crafts catalysts (e.g., zinc bromide or ferric bromide). The bromo precursor of ketoprofen can be prepared by bromination of methyl benzoate (or a similar lower hydrocarbon ester) using aluminum chloride followed by NaOH hydrolysis, conversion to the acid chloride (e.g., with SOCl₂) and reaction with benzene (again, using a Friedel-Crafts catalyst such as AlCl₃). The precursor for (±)-2-(6-methoxy-2-naphthyl)propionic acid, viz. , 2-bromo-6-methoxynaphthalene, is best made by the process described in commonly-owned U.S. application Serial No. 08/780,309, filed January 8, 1997.

In addition to the profen compounds described above, other profen compounds which can be prepared under appropriate conditions by use of this invention to convert the corresponding bromo precursors by reaction with ethylene include protizinic acid, tiaprofenic acid, indoprofen, benoxaprofen, carprofen, pirprofen, pranoprofen, alminoprofen, suprofen and loxoprofen.

The following examples are given to illustrate the process of this invention and are not intended as a limitation thereof. Unless otherwise specified all parts and percentages are by weight.
The following designations are used in the examples:
BMN is 2-bromo-6-methoxynaphthalene.
TEA is triethylamine.
DEK is diethyl ketone.
NMDP is neomenthyldiphenylphosphine.
MVN is 6-methoxy-2-vinylnaphthalene.
THF is tetrahydrofuran.
ACN is acetonitrile. As is well known in the art, the terms or designations "racemic 2-(6-methoxy-2-naphthyl)propionic acid and "(±)-2-(6-methoxy-2-naphthyl)propionic acid" mean exactly the same thing. For convenience, "sodium racemate" is sometimes used in the examples to refer to racemic sodium 2-(6-methoxy-2-naphthyl)propionate.

Example 1 illustrates a preferred overall procedure for producing racemic 2-(6-methoxy-2-naphthyl) propionic acid on a large (1000 gallon) scale using fresh DEK.

### EXAMPLE 1

### Arylation Reaction

To a 1000 gallon reactor are charged 750 kg of BMN, 1305 kg of DEK, 368 kg of TEA, 0.3 kg of PdCl₂, 3.1 kg of NMDP, and 37 kg of water. The reactor is sealed, pressured to 100 psig with ethylene and the reactor temperature is adjusted to 95°C. The reactor is then pressured to 425-450 psig with ethylene and held at this pressure until the uptake of ethylene is completed. The reactor is cooled to 60°C and excess ethylene is vented from the reactor. The reaction typically takes 4-6 hours to go to completion and typically gives a > 95 % BMN conversion and a MVN yield of 85-95%.

### Product Workup and Solvent Exchange

To the reaction product from the arylation reaction is added 557 kg of a 25 wt% aqueous sodium hydroxide solution. The mixture is stirred for 15 minutes at 50-60°C and then allowed to stand for 15 minutes. The bottom aqueous solution is drained from the vessel. The organic phase is then subjected to distillation at reduced pressure, typically in the range of 200 mm Hg to 350 mm Hg to distill off TEA to a level at which the weight ratio of TEA:MVN is less than 0.016. After adding THF to the residual organic phase (distilland or pot residue) to form a mixture in which the THF:DEK weight ratio is approximately 1:1, this mixture is filtered to remove solids (palladium catalyst residues and oligomeric or dimeric coproduct).

### Hydrocarboxylation Reaction

Charged to a 1000 gallon reactor are a filtered THF-DEK-MVN solution produced as in the above workup procedure containing 550 kg of MVN, 825 kg of DEK, and 825 kg of THF, followed by 0.3 kg of PdCl₂, 0.64 kg of CuCl₂, 3.1 kg of NMDP, and 200 kg of 10 wt% HCl. The reactor is then pressured to 100 psig with carbon monoxide and the reactor temperature is adjusted to 70°C. The reactor is then pressured to 360 psig with carbon monoxide and held at this pressure until the uptake of carbon monoxide is completed. The reactor is then cooled and the pressure is vented. The reaction typically takes 4-8 hours to go to completion with > 95% MVN conversion and a yield of racemic 2-(6-methoxy-2-naphthyl)propionic acid of 90%.

### Racemic Product Workup and Recovery

Aqueous sodium hydroxide (25 wt% solution) is added to the reactor to convert the racemic 2-(6-methoxy-2-naphthyl)propionic acid to racemic sodium 2-(6-methoxy-2-naphthyl)propionate, and to neutralize the HCl remaining in the reaction mixture. The THF is then distilled from the reaction mixture at atmospheric pressure. (These neutralization and distillation steps can be reversed if the materials of construction of the reactor overhead are resistant to HCl). The resultant aqueous phase is separated from the organic phase which is composed mainly of DEK and impurities. The residual organics (e.g., DEK) contained in the aqueous phase are distilled from the aqueous racemic sodium 2-(6-methoxy-2-naphthyl)propionate phase at atmospheric pressure. This sodium racemate solution is desirably a 10-35 wt% solution, and if necessary, the concentration is adjusted to fall in this range by removal or addition of water. The aqueous sodium racemate phase is then washed with toluene to remove neutral impurities. Typically one to three toluene washes, preferably at least two, are used. A suitable temperature, typically 60-80°C. is maintained to prevent the racemic sodium 2-(6-methoxy-2-naphthyl)propionate from precipitating. The aqueous solution is then acidified with sulfuric acid in the presence of toluene at 97°C. The aqueous phase is cut from the bottom of the reactor and the toluene solution of (±)-2-(6-methoxy-2-naphthyl)propionic acid is washed with water (typically twice) at 95°C to remove residual sulfuric acid. Racemic 2-(6-methoxy-2-naphthyl)propionic acid is then crystallized from the toluene solution.

Example 2 illustrates a preferred overall procedure for producing racemic 2-(6-methoxy-2-naphthyl)propionic acid on a large (1000 gallon) scale using recycle solvent (principally DEK and TEA) from a process conducted as in Example 1 above.

### EXAMPLE 2

To a 1000 gallon reactor are charged 750 kg of BMN, a mixture of recycle solvent (DEK and TEA mixture containing typically 1 wt% water) to give approximately 1305 kg of DEK and 368 kg of TEA. Catalyst consisting of 0.3 kg of PdCl₂, and 3.1 kg of NMDP is charged to the reactor. Fresh water is added (if necessary) to raise the water content of the reaction mixture to approximately 1.6 wt%. The reactor is then pressured to 100 psig with ethylene and the reactor temperature is adjusted to 95°C. The reactor is then pressured to 425-450 psig with ethylene and held at this pressure until the uptake of ethylene is completed. The reactor is cooled to 60°C and excess ethylene is vented from the reactor. The reaction typically takes 4-6 hours to go to completion and typically gives a >95% BMN conversion and a MVN yield of 85-95%.

Aqueous caustic (25% aqueous NaOH solution) is added to the reaction mixture containing MVN to liberate the TEA from the triethylamine hydrobromide salt. The aqueous layer is then separated from the organic layer, and the TEA is then recovered from the MVN, DEK, and TEA mixture by distillation. The distillate composed of DEK, TEA, and water is then recycled for use in the arylation reaction. THF is added to the distillation residue (distilland or pot residue) composed mainly of a MVN/DEK mixture plus some solids to produce a MVN mixture containing THF and DEK in a weight ratio of 1:1 suitable for carboxylation. The resultant mixture is filtered to remove the solids therefrom. Fresh catalyst and HCl are added in proportions corresponding to those of Example 1 and the hydrocarboxylation reaction is carried out as in Example 1. Then the (±)-2-(6-methoxy-2-naphthyl)propionic acid is converted to sodium (±)-2-(6-methoxy-2-naphthyl)propionate by the addition of 25 wt% aqueous sodium hydroxide solution. and the remainder of the racemic product workup and recovery procedure of Example 1 is carried out.

The procedure of Example 1 above can be conducted in the same manner except for the omission of the reaction accelerating amount of water in the arylation reaction. The reaction proceeds, but proceeds more slowly than if the water is present in the arylation reaction. This is illustrated in Example 3 hereof.

### EXAMPLE 3

A series of 12 arylation runs was conducted in a 2-liter reactor in which the proportions of the ingredients and the reaction conditions used were, except for some small inconsequential differences, the same from run to run, the only independent variable being water content and the amount thereof. The reaction mixtures were composed of 300 g of BMN, 529-530 g of DEK, 147-148 g of TEA, 0.112-0.116 g of PdCl₂, and 1.23-1.26 g of NMDP. Several runs were conducted with no added water, and the remainder had measured quantities of added water. All reactions were performed at 95°C under ethylene at 420 to 450 psig. The criterion for reaction rate was maximum rate of ethylene consumption during each reaction. Thus the higher this value, the better. The results of these runs as regards reaction rates are summarized in the table.

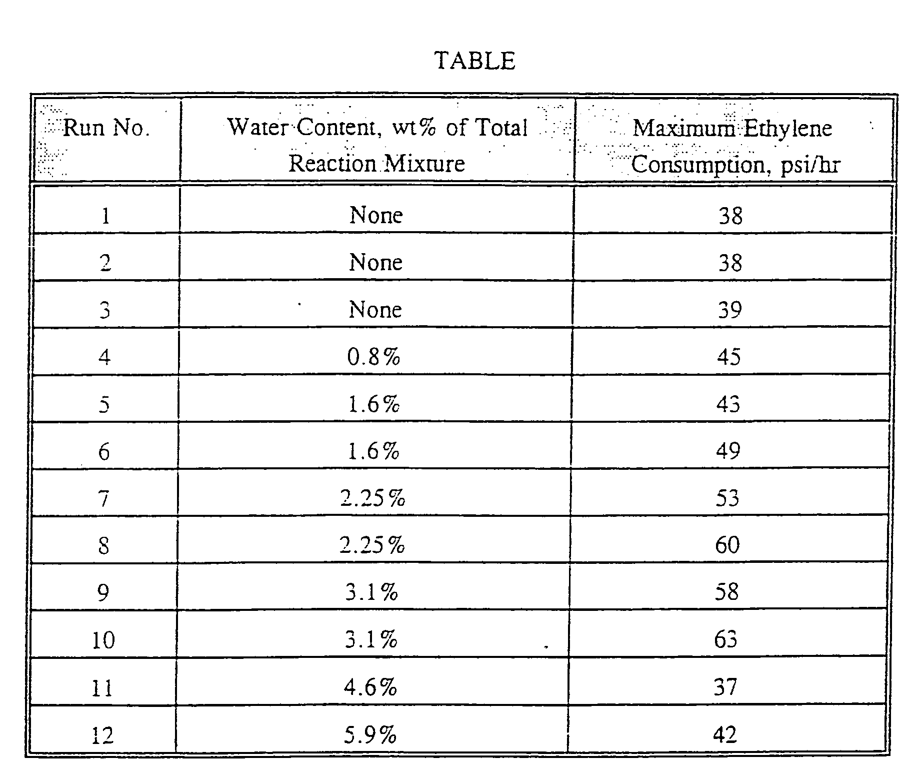

The inclusion or presence of small amounts of water in the range of 0.5 to 5 percent of the total weight of the arylation reaction mixture as a reaction accelerator in the arylation reaction is in accordance with the subject matter of, and as disclosed in full in, commonly-owned copending application Serial No. 08/780,310, filed January 8, 1997.

Experimental work has shown that it is advantageous to carry out the separation of solids from the arylation reaction product after the separation of the free amine and the replenishment of the solvent by addition of THF or like solvent (as in Examples 1 and 2), rather than before such separation and solvent addition. In particular, the filtration time is reduced significantly in this manner.

As noted above, if in the arylation reaction more than one solvent/diluent is used, the amine does not have to boil below all such solvent/diluents. Instead it should boil below at least one of the solvent/diluents that makes up a substantial portion (e.g., at least 20 or 30%) of the total weight of such solvent/diluents. For example, a reaction conducted generally as in Example 1 above using a 1:1 (wt:wt) mixture of acetonitrile (ACN) and diethyl ketone (DEK) as the solvent/diluents, involves a situation in which the triethylamine boils above the ACN, but below the DEK. In such case, different workup procedures can be used. In one such procedure the ACN can be distilled (stripped) from the reaction mixture, and then the aqueous inorganic base solution is added followed by the phase separation and distillation of the triethylamine from the remaining organic phase. Another procedure involves adding the aqueous inorganic base solution, conducting the phase separation, and then distilling off the ACN and the triethylamine, leaving the diethyl ketone solution behind.

Additional examples of the practice of this invention are given below.

### EXAMPLE 4

### Preparation of 6-Methoxy-2-Vinylnaphthalene

A 20-gallon jacketed stainless steel reactor equipped with a mechanical agitator is charged with 19.45 kg of acetonitrile (ACN) and 12.45 kg of 2-bromo-6-methoxynaphthalene (BMN), and 4.8 g of PdCl₂. The reactor is pressured and vented three times with 50 psig nitrogen. The reactor is then charged with 5.3 kg of ACN and 5.64 kg of triethylamine (TEA). The agitator is set at 158 rpm and the reactor is pressured and vented three times with 80 psig nitrogen. The reactor is then purged for ten minutes with nitrogen. Next a mixture of 48.6 g of neomenthyldiphenylphosphine (NMDP) dissolved in 0.35 kg of TEA is charged to the reactor. The agitator is set to 412 rpm and the reactor is heated with steam on the jacket. The reaction temperature is initially in the range of 91-109°C, while the pressure varies from 412-519 psig. The reaction produces a heat kick, and after 30 minutes the temperature rises to 109°C with 26°C cooling water on the jacket. The total reaction time is 1.75 hours with a BMN conversion of 100%. The reactor is cooled, vented, and the reactor contents are transferred to a 30-gallon glass lined reactor for workup.

### Workup of 6-Methoxy-2-Vinylnaphthalene

The crude 6-methoxy-2-vinylnaphthalene (MVN) solution in the 30-gallon reactor is stripped at 330 mm Hg to remove the ACN. The total strip time is 6.33 hours with a maximum bottoms temperature of 91°C. The final overhead temperature is 68°C. Zero reflux is used for the first 35 minutes of operation. The reflux ratio is then set to five, and 34.95 kg of diethyl ketone (DEK) is added to the reactor contents. The reflux ratio remains at five for the duration of the strip.

After charging 9.25 kg of 25% NaOH to the stripped reaction product in the 30-gallon reactor, the resultant mixture is agitated for 30 minutes. Then the agitator is shut off and the aqueous phase is allowed to settle for 1.75 hours. The mixture is phase cut at 57°C, and the aqueous phase is collected and discarded. The organic phase and rag layer in the reactor are stripped to remove TEA. The strip pressure is 330 mm Hg. The total strip time is 4.9 hours. The column is started up under total reflux for the first 30 minutes of operation. The reflux ratio is then lowered to three for 3.5 hours. The reflux ratio is reduced to two for the remainder of the strip. The final overhead temperature is 79°C and the final bottoms temperature is 86°C.

To the cooled-down stripped mixture in the 30-gallon reactor is added 8 kg of tetrahydrofuran (THF). The resultant MVN solution is filtered through a 10 micron bag filter and a 1 micron cartridge filter.

### Hydrocarboxylation of 6-Methoxy-2-Vinylnaphthalene

A 20-gallon Hastalloy reactor is purged three times with 80 psig nitrogen, and then 3.8 g of PdCl₂ and 8.8 g of CuCl₂ are charged to the reactor, followed by the MVN solution. The reactor is purged three more times with 80 psig nitrogen and the agitator is set to 118 rpm. After charging 3.6 kg of THF and 3.55 kg of 10% HCl to the reactor, the reactor is again purged three times with 80 psig nitrogen and then nitrogen is bubbled through a dip leg for ten minutes. Next, a mixture of 42.2 g of NMDP and 0.35 kg of THF is charged to the reactor and the agitator is set at 402 rpm. The reactor is pressured and vented three times with 50 psig CO, and then heated to reaction temperature and pressured with CO. The reaction temperature is in the range of 70 to 78°C, while the pressure varies from 247 to 450 psig. After a total reaction time of 8.5 hours the reactor is cooled and vented, and the contents transferred to a 30-gallon glass-lined reactor for workup.

### Product Workup

The hydrocarboxylation mixture is neutralized with 2.05 kg of 25% NaOH. THF is stripped at atmospheric pressure from the workup reactor contents over 2.5 hours. Water (30.7 kg) is charged 1.4 hours into the strip. The final overhead temperature is 97°C and the final bottoms temperature is 108°C. To the stripped reactor contents is added 7 kg of 25% NaOH, and the mixture is agitated for 30 minutes at 50-60 °C. After a 35-minute settling time, the aqueous and organic phases are separated from each other. The aqueous phase is charged back to the workup reactor along with 10 kg of toluene. This mixture is agitated for 15 minutes and allowed to settle for 30 minutes at 55°C. The phases are again separated. The aqueous phase is charged back to the workup reactor along with 10 kg of toluene. the mixture is stirred for 15 minutes and then allowed to settle. The mixture is then heated to 65°C and the phases are separated from each other. The aqueous phase is again charged back to the reactor along with 10 kg of toluene. The mixture is stirred for 15 minutes and allowed to settle for 30 minutes at 70°C, and a final phase cut is made. The separated aqueous phase is a clear amber aqueous solution of sodium (±)-2-(6-methoxy-2-naphthyl)propionate.

### EXAMPLE 5

The procedure of Example 4 is repeated substantially as described with the following principal changes:

The initial charge to the first reactor is 21.4 kg of diethyl ketone (DEK), 12.4 kg of BMN, and 4.6 g of PdCl₂. The second charge is 3.2 kg of DEK and 6.34 kg of TEA. The 10-minute nitrogen purge after the addition of the TEA addition is eliminated. The NMDP charge (50.9 kg) is added as a solution in 0.27 kg of DEK. The pressurizing with ethylene is started to 100 psig before beginning the heat up of the reactants. This arylation reaction is conducted at 92-98°C and 393-429 psig.

The MVN workup involves addition of 10.15 kg of DEK, heating to 75°C, followed by the caustic wash, a phase cut, a water wash, another phase cut, and the TEA strip with a final overhead temperature of 79°C and a maximum bottoms temperature of 97°C.

The hydrocarboxylation solvent is a mixture of residual DEK and 8.2 kg of added THF. The other components charged are 3.5 g of PdCl₂, 7.9 g of CuCl₂, 3.25 kg of 10% HCl, 37.9 g of NMDP in 160 g of DEK. The hydrocarboxylation reaction is performed for 8. 7 hours, with temperatures in the range of 74 to 84°C and pressures in the range of 321 to 476 psig.

The crude (±)-2-(6-methoxy-2-naphthyl)propionic acid is stripped of THF, converted to sodium (±)-2-(6-methoxy-2-naphthyl)propionate and washed three times with 5 kg of toluene to yield an aqueous solution of sodium (±)-2-(6-methoxy-2-naphthyl)propionate.

### EXAMPLE 6

### Preparation of 6-Methoxy-2-Vinylnaphthalene

A 20-gallon jacketed stainless steel reactor equipped with a mechanical agitator is charged with 12.8 kg of ACN, 12.45 kg of DEK and 12.4 kg of 2-bromo-6-methoxynaphthalene (BMN), 4.6 g of PdCl₂, and 50.9 g of NMDP. The reactor is pressured and vented three times with 50 psig nitrogen. The reactor is then charged with 6.27 kg of TEA. The agitator is set at 158 rpm and the reactor is pressured and vented with 50 psig nitrogen. The agitator is set to 416 rpm, the reactor is pressured to 100 psig with ethylene and heated with tempered water on the jacket. The reaction temperature ranges from 87 to 98°C, while the pressure varies from 394 to 458 psig. The total reaction time is 3.5 hours with a BMN conversion of 99.6 % in two hours. The reactor is cooled, vented, and the reactor contents at 60°C are transferred for workup, to a 30-gallon glass lined reactor equipped with a 6-inch column. The 20-gallon reactor is then charged with 12.5 kg of DEK, which is then heated to 60°C and transferred to the 30-gallon reactor.

### Workup of 6-Methoxy-2-Vinylnaphthalene

The crude 6-methoxy-2-vinylnaphthalene (MVN) solution in the 30-gallon reactor is stripped at 150 mm Hg to remove the ACN . The total strip time is 4 hours with a maximum bottoms temperature of 73°C. The final overhead temperature is 59°C. Reflux ratios used are 5:1 for 1.9 hours, 3:1 for 1.6 hours, and 4:1 for 1.5 hours.

After charging 9.3 kg of 25% NaOH to the stripped reaction product in the 30-gallon reactor, the resultant mixture is agitated for 15 minutes at 35°C. Then the agitator is shut off and the aqueous phase is allowed to settle for 30 minutes. The mixture is phase cut and the organic phase is washed in the reactor with 1.2 kg of water with stirring for 15 minutes. After allowing a settling period of 30 minutes, another phase cut is made. A TEA strip of the organic phase is conducted at 150 mm Hg. The total strip time is 5.25 hours. The highest overhead temperature is 59°C and the maximum bottoms temperature is 91°C. The reflux ratios were 50:1 at start up, and when the column stabilized, the reflux ratio was reduced to 5:1 for 2.25 hours and 7:1 for the final 2.5 hours of the strip. The reaction product is then diluted by addition to the reactor of 12.05 kg of THF and 2.05 kg of DEK. The resulting solution is then filtered through a ten-micron bag filter and a one-micron cartridge filter.

### Hydrocarboxylation of 6-Methoxy-2-Vinylnaphthalene

The filtered MVN solution is charged to a 20-gallon Hastalloy reactor followed by an additional 4.65 kg of DEK. Then 4.6 g of PdCl₂ and 10.5 g of CuCl₂ are charged to the reactor. The reactor is purged three times with 50 psig nitrogen, and 4.2 kg of 10% Hcl is charged. The reactor is pressured to 80 psig with nitrogen and vented. A solution of 50.9 g of NMDP in 255 g of DEK is charged to the reactor and the reactor is pressured and vented twice with 50 psig nitrogen with the agitator running only when pressurizing. The agitator speed is set at 399 rpm and the reactor is pressured and vented three times with 50 psig CO, again agitating only during pressurization. The reactor is then pressured to 280 psig with CO and heated to 75°C. The reaction temperature is kept in the range of 73 to 77°C, while the pressure varies from 339 to 350 psig. After a total reaction time of 6 hours the reactor is cooled and vented, and the contents transferred to a 30-gallon glass-lined reactor for workup.

### Product Workup

The hydrocarboxylation mixture is neutralized with 2.15 kg of 25 % NaOH. THF is stripped from the hydrocarboxylation mixture at atmospheric pressure over 1.2 hours. The final bottoms temperature is 100°C and the final overhead temperature is 92°C. Water (30.7 kg) is charged 1.4 hours into the strip. The final overhead temperature is 97°C and the final bottoms temperature is 108°C. DEK (4.95 kg) is added to the stripped reactor contents, followed by 14 kg of water and 7.55 kg of 25% NaOH, and the mixture is agitated for 30 minutes at 70-80°C. After a 30-minute settling time, the aqueous and organic phases are separated from each other. The aqueous phase is charged back to the workup reactor and stripped of DEK with a final bottoms temperature of 95°C and a final overhead temperature of 95°C. A 2.0 kg water charge is added along with 5.15 kg of toluene. This mixture is agitated for 20 minutes and allowed to settle overnight with 60°C tempered water in the jacket. The phases are then separated. The aqueous phase is washed two more times with toluene (the first time with 5.1 kg, the second time with 4.95 kg) each time followed by a phase separation. The product is recovered as a water solution of sodium (±)-2-(6-methoxy-2-naphthyl)-propionate.

### EXAMPLE 7

### Preparation of 6-Methoxy-2-Vinylnaphthalene

The 20-gallon jacketed stainless steel reactor is charged with a 12.5 kg of ACN, 12.5 kg of methyl isobutyl ketone (MIBK), and 12.45 kg of BMN, 4.6 g of PdCl₂, and 50.9 g of NMDP. The reactor is pressured and vented three times with 50 psig nitrogen. Then 6.8 kg of TEA is charged. The agitator is set at 160 rpm and the reactor is pressured and vented with 50 psig nitrogen. The agitator is set to 415 rpm, the reactor is pressured to 100 psig with ethylene, and heated with tempered water on the jacket. The reaction temperature ranges from 94 to 100°C, while the pressure varies from 388 to 432 psig. The total reaction time is 2.6 hours, but the reaction reaches 99% conversion in 1.8 hours. The reactor is cooled and the ethylene pressure is vented. After standing for 16 hours with the agitator in operation, the reactor is heated to approximately 60°C and the reactor contents are transferred to the 30-gallon glass-lined workup reactor. The 20-gallon reactor is charged with 12.4 kg of MIBK, which is then heated to 60°C and also transferred to workup reactor.

### Workup of 6-Methoxy-2-Vinylnaphthalene

The crude MVN solution is stripped at 150 mm Hg to remove the ACN. The total strip time is 3.3 hours with a maximum bottoms temperature of 76°C. A reflux ratio of 50 is used to line out the column. After the column stabilizes, the reflux ratio is reduced to five. This reflux ratio is maintained for 45 minutes and then reduced to three for 30 minutes. The reflux ratio is set at two for the next 55 minutes before finally switching to zero reflux for the last 25 minutes.

After cooling to 47°C, 9.4 kg of 25% NaOH is charged to the stripped mixture. The temperature drops with the addition of the caustic. The reactor is agitated for 15 minutes and then the agitator is shut off and the aqueous phase is allowed to settle for 30 minutes. The phases are separated, and a 1.05 kg water wash is charged to the organic phase and mixed therewith for 20 minutes. This is allowed to settle for 80 minutes and the aqueous phase is cut from the bottom of the reactor.

The TEA strip pressure is initially 150 mm Hg and is lowered throughout the strip to a final value of 70 mm Hg. The total strip time is 4.25 hours with a maximum bottoms temperature of 78°C. The column is started up with a zero reflux ratio for the first 35 minutes of operation. The reflux ratio is then set at five and held there for 25 minutes. The reflux ratio is decreased to two for the final 3.25 hours of the strip. To the stripped product mixture is charged 8.1 kg of THF and the resultant MVN solution is filtered through a ten micron bag filter and a one micron cartridge filter. An additional 4.05 kg of THF is charged to the workup reactor and this is also filtered.

### Hydrocarboxylation of 6-Methoxy-2-Vinylnaphthalene

The MVN solution is transferred to the above hydrocarboxylation reactor. To this are charged 4.3 g of PdCl₂ and 9.8 g of CuCl₂. The reactor is purged once with 50 psig nitrogen. The agitator is set to 118 rpm and 3.95 kg of 10% HCl is charged. The reactor is pressured to 80 psig with nitrogen and vented twice (agitating during pressurization, no agitation during the vent). A solution of 47.6 g NMDP in 248 g DEK is charged. The agitator speed is set at 401 rpm and the reactor is pressured and vented three times with 50 psig CO (agitating during pressurization. no agitation during the vent). The reactor is then pressured to 276 psig with CO and heated to 75°C. The reactor temperature varies from 72 to 80°C and the pressure range is 334 to 355 psig. The reaction is shutdown after 8.8 hours.

### Product Workup

The (±)-2-(6-methoxy-2-naphthyl)propionic acid solution is charged to a workup reactor and neutralized with 2.0 kg of 25% NaOH. THF is stripped at atmospheric pressure over 20 minutes. The final bottoms temperature is 79°C and the final overhead temperature is 77°C. The stripped mixture is cooled to 60°C and to this are charged 14.0 kg of water and 8.0 kg of caustic. The mixture is agitated for 30 minutes at 75 °C. The agitator is shut off and the contents of the reactor are allowed to settle for 30 minutes. The phases are separated. The aqueous solution is charged back to the reactor and left agitating for 16 hours. The aqueous solution is then stripped at atmospheric pressure for 1.5 hours. The aqueous phase in the column is cut back to the reactor. One more strip is done using steam on the jacket. Additional distillate is drained from the column following the strip. The final bottoms temperature for the strip is 101°C and the final overhead temperature is 100°C. A 5.05 kg charge of toluene is added to stripped product mixture, and the mixture is agitated for 20 minutes at 68°C then allowed to settle for 30 minutes. The phases are cut to give an amber-orange aqueous solution and a dark-green organic solution. The aqueous solution is washed with 5.0 kg of toluene, giving a reddish-purple clear aqueous solution and a cloudy olive-green organic solution. The third toluene wash (5.05 kg, 71°C) produces a clear purple aqueous solution and a cloudy yellow organic solution.

### EXAMPLE 8

The procedure of Example 7 is repeated substantially as described with the following principal changes:

The initial charge to the first reactor is 12.4 kg of ACN, 12.65 kg of DEK, 12.45 kg of BMN made as in Example 7 hereof, 4.6 g of PdCl₂, and 51 g of NMDP. The second charge is 6.17 kg TEA. This 2.5-hour arylation reaction is conducted at 88-99°C and 318-458 psig.

The ACN distillation in the MVN workup is at 150 mm Hg and involves a total strip time of 5.25 hours with a maximum bottoms temperature of 71.8°C. The TEA strip pressure is initially 150 mm Hg and is lowered throughout the 4-hour strip to a final value of 90 mm Hg.

The hydrocarboxylation solvent is a mixture of residual DEK and 12 kg of added THF. The other components charged are 4.1 g of PdCl₂, 9.2 g of CuCl₂, 3.65 kg of 10% HCl, 44.7 g of NMDP in 222 g of DEK. The hydrocarboxylation reaction runs for 6.6 hours, with temperatures in the range of 74 to 77°C and pressures in the range of 333 to 358 psig.

As in Example 7, the crude (±)-2-(6-methoxy-2-naphthyl)propionic acid is converted to sodium (±)-2-(6-methoxy-2-naphthyl)propionate, stripped of THF, and washed three times, each time with 5 kg of toluene, to yield an aqueous solution of sodium (±)-2-(6-methoxy-2-naphthyl)propionate.

### EXAMPLE 9

The procedure of Example 7 is repeated substantially as described with the following principal changes:

The initial charge to the first reactor is 12.55 kg of ACN, 12.5 kg of MIBK, 12.5 kg of BMN made as in Example 7 hereof. 4.6 g of PdCl₂, and 51 g of NMDP. The second charge is 6.19 kg TEA. This 2.7-hour arylation reaction is conducted at 88-97°C and 371-441 psig.

The ACN distillation in the MVN workup is at 150 mm Hg and involves a total strip time of 3.8 hours with a maximum bottoms temperature of 71°C. The TEA strip pressure is initially 150 mm Hg and is lowered throughout the 5.3-hour strip to a final value of 70 mm Hg.

The hydrocarboxylation solvent is a mixture of residual MIBK and 12 kg of added THF. The other components charged are 4.6 g of PdCl₂, 9.5 g of CuCl₂, 3.85 kg of 10% HCl, 47 g of NMDP in 226 g of DEK. The hydrocarboxylation reaction is conducted for 7 hours, with temperatures in the range of 72 to 77°C and pressures in the range of 333 to 357 psig.

As in Example 7, the crude (±)-2-(6-methoxy-2-naphthyl)propionic acid is converted to sodium (±)-2-(6-methoxy-2-naphthyl)propionate, stripped of THF, and washed three times, each time with 5 kg of toluene, to yield an aqueous solution of sodium (±)-2-(6-methoxy-2-naphthyl)propionate.

Example 10 illustrates a preferred procedure for producing BMN starting material for use in the practice of this invention.

### EXAMPLE 10

### Bromination of 2-Naphthol

2-Naphthol (144.8 g, 1.00 mol), EDC (537 g), and water (162 g) are charged to a 2-L reactor equipped with a reflux condenser, mechanical stirrer and peristaltic pump addition system. The reactor is heated to 55°C until most of the β-naphthol is dissolved. Bromine (336.9g, 2.11 mol) is then added (sub-surface) via a pump at such a rate so as to maintain the reaction temperature at 60°C. After the bromine addition, the reaction temperature is maintained at 60°C for 1.5 hour. The reaction is then cooled slightly and the lower phase (aq. HBr) siphoned off. The remaining EDC solution (841 g) is transferred out of the reactor and analyzed by GC. In a run conducted in this manner, the analysis showed 0.4% 2-naphthol, 92.6 % 1,6-dibromo-2-naphthol (DBN). and 4.9% of other isomers.

### Hydrodebromination of 1,6-Dibromo-2-Naphthol

A solution of DBN (271 g, 0.9 mol) in ethylene dichloride (EDC) (551 g), obtained from the bromination reaction, is charged in a 1000 mL Hastalloy B autoclave. Tungsten carbide (82 g, 30 wt%) and tetrabutylammonium bromide (0.2 g, 0.1 wt%) are added and the reactor is sealed. The reactor is purged with hydrogen (50 psig) and vented three times and then pressured with hydrogen and heated to 90°C. A constant purge of hydrogen is maintained in such a rate that the pressure remains in the 120-125 psig range. Analysis of a reaction mixture produced after 5.5 hours in this manner showed 90% 6-bromo-2-naphthol. 2% DBN, and 2% 2-naphthol. The reactor is cooled to room temperature, vented to scrubbers. and the catalyst is permitted to settle. The EDC solution (747 g in a reaction conducted in this manner) is removed through the dip tube.

### Methylation of 6-Bromo-2-Naphthol with MeCl

The EDC solution formed as above is transferred to a 1.4-liter (three pints) Chemco glass reactor with stainless steel head. It is first neutralized with dilute acid and then concentrated by distillation. Water (50 mL) is added to azeotropically remove traces of EDC left in the residue. Isopropyl alcohol (242 g) and sodium hydroxide (44 g, 1.1 mol; 88 g of 50% solution) are charged into the reactor. The reactor is sealed, purged with nitrogen, and heated to 70°C. Methyl chloride (MeCl) (66 g, 1.3 mol) is charged over a period of one hour (40-50 psig). After stirring at 80°C for another hour, isopropyl alcohol is removed by distillation. The residue is heated to melted condition (90-95°C) and then it is washed with water (400 g). Water is removed and the residue is distilled under vacuum (1 mm Hg). After removing small amounts of volatile materials. BMN is distilled at 160-165°C as a white solid (169 g was formed in an operation conducted in this manner). Isopropyl alcohol (490 g) is added and the solution was heated to reflux and then slowly cooled down to 10°C. Solid BMN is removed and washed with cold (0°C) isopropyl alcohol (180 g) and then dried under vacuum at 70-75°C. Analysis of the white crystalline product formed in this manner showed 99.7 wt% BMN.

Example 10 involves procedures and subject matter described in full in commonly-owned copending application Serial No. 08/780.309, filed January 8, 1997.

Example 11 illustrates the dilute acid wash workup procedure for separating the arylation product and the secondary or tertiary amine use as the hydrogen halide acceptor in the arylation reaction.

### EXAMPLE 11

To a 5 gallon stainless steel magnetically stirred autoclave are added DEK (3296 g), BMN (1502 g, 6.34 mol), NMDP (6.170 g, 19.0 mmol), PdCl₂ (0.574 g, 3.2 mmol), and TEA (684 g, 6.76 mol). The reactor is purged with nitrogen and then filled with ethylene and heated to 95°C. The reaction mixture is stirred for 4.5 hours at 95°C under ethylene pressure (609 to 640 psig) and then cooled to 60°C and slowly vented. The reactor is emptied yielding a mixture of yellow solids and yellow liquid. A 5000 g portion of this reaction mixture is poured into a 12 L flask fitted with a mechanical stirrer and a bottom outlet. Water (695 g) and 10 wt% aqueous HCl (209 g) are added to the reaction mixture, and the mixture is stirred and warmed to 65°C. and then allowed to stand and settle. The aqueous phase is removed from the yellow organic phase. The organic phase is washed a second time with a mixture of water (250 g) and 10 wt% aqueous HCl at 64°C, and the aqueous phase is separated from the washed organic phase which is composed mainly of MVN and DEK. The washed organic phase is hydrocarboxylated as described above, preferably after stripping off some of the DEK and replacing it with THF. The aqueous phases contain triethylamine hydrochloride, from which TEA can be recovered by addition of a strong base such as aqueous NaOH, followed by a phase separation.

It is to be understood that the reactants and components referred to by chemical name or formula anywhere in the specification or claims hereof, whether referred to in the singular or plural, are identified as they exist prior to coming into contact with another substance referred to by chemical name or chemical type (e.g., another reactant, or a solvent). It matters not what preliminary chemical changes, transformations and/or reactions, if any, take place in the resulting mixture or solution or reaction medium as such changes, transformations and/or reactions are the natural result of bringing the specified reactants and/or components together under the conditions called for pursuant to this disclosure. Thus the reactants and components are identified as ingredients to be brought together in connection with performing a desired chemical reaction or in forming a mixture to be used in conducting a desired reaction. Accordingly, even though the claims hereinafter may refer to substances, components and/or ingredients in the present tense ("comprises", and/or "is"), the reference is to the substance, component or ingredient as it existed at the time just before it was first contacted, blended or mixed with one or more other substances, components and/or ingredients in accordance with the present disclosure. Without limiting the generality of the foregoing, as an illustrative example, where a claim specifies that a catalyst is a palladium compound in combination with a tertiary phosphine ligand, this phraseology refers to the makeup of the individual substances before they are combined and/or mixed separately or concurrently with one or more other materials, and in addition. at the time the catalyst is actually performing its catalytic function it need not have its original makeup -- instead whatever transformations, if any, that occur *in situ* as the catalytic reaction is conducted is what the claim is intended to cover. Thus the fact that a substance, component or ingredient may have lost its original identity through a chemical reaction or transformation during the course of contacting, blending or mixing operations, if conducted in accordance with this disclosure and with the application of common sense and the ordinary skill of a chemist, is thus wholly immaterial for an accurate understanding and appreciation of the true meaning and substance of this disclosure and the claims thereof.

This invention is susceptible to considerable variation in its practice. Therefore the foregoing description is not intended to limit, and should not be construed as limiting, the invention to the particular exemplifications presented hereinabove. Rather, what is intended to be covered is as set forth in the ensuing claims and the equivalents thereof permitted as a matter of law.

## Claims

1. A process which comprises (a) forming a reaction product composition comprising arylolefin or substituted arylolefin and amine-hydrohalide in a liquid polar organic solvent medium by palladium-catalyzed arylation of vinylolefin with an aryl halide and/or substituted aryl halide in a liquid polar organic solvent containing one or more secondary or tertiary amines as hydrogen halide acceptor and (b) mixing with at least aportion of said reaction product composition a dilute aqueous acid to thereby form (i) an organic phase containing said arylolefin or substituted arylolefin, and (ii) an acidic aqueous phase containing dissolved amine hydrohalide, and (c) separating at least a portion of said phases from each other.

2. A process according to Claim 1 wherein in (a) the aryl halide and/or substituted aryl halide is an aryl bromide or substituted aryl bromide, and wherein said dilute aqueous acid is dilute aqueous hydrochloric acid.

3. A process according to Claim 1 further comprising subjecting at least a portion of the separated organic phase from (c) to palladium-catalyzed carboxylation to form arylcarboxylic acid or ester or substituted arylcarboxylic acid or ester.

4. A process according to Claim 3 wherein the palladium-catalyzed carboxylation is palladium-catalyzed hydrocarboxylation to form arylcarboxylic acid or substituted arylcarboxylic acid.

5. A process according to Claim 4 further comprising mixing together at least a portion of the separated aqueous phase from (c) and a strong inorganic base to form free amine and an aqueous solution of inorganic halide.

6. A process according to Claim 5 wherein the aryl halide and/or substituted aryl halide is an aryl bromide or substituted aryl bromide.

7. A process according to Claim 4 wherein said aryl bromide or substituted aryl bromide is 2-bromo-6-methoxynaphthalene.

8. A process according to Claim 7 wherein said one or more secondary or tertiary amines consist essentially of triethylamine, and wherein said dilute aqueous acid is dilute aqueous hydrochloric acid.

9. A process according to Claim 5 wherein said aryl bromide or substituted aryl bromide is 2-bromo-6-methoxynaphthalene.

10. A process according to Claim 9 wherein said one or more secondary or tertiary amines consist essentially of triethylamine, and wherein said dilute aqueous acid is dilute aqueous hydrochloric acid.

11. A process according to Claim 4 wherein said aryl bromide or substituted aryl bromide is 4-bromoisobutylbenzene.

12. A process according to Claim 11 wherein said one or more secondary or tertiary amines consist essentially of triethylamine, and wherein said dilute aqueous acid is dilute aqueous hydrochloric acid.

13. A process according to Claim 5 wherein said aryl bromide or substituted aryl bromide is 4-bromoisobutylbenzene.

14. A process according to Claim 13 wherein said one or more secondary or tertiary amines consist essentially of triethylamine, and wherein said dilute aqueous acid is dilute aqueous hydrochloric acid.
